# EUROPEAN PATENT APPLICATION

(11) **EP 3 006 941 A1**
(43) Date of publication of application: **13.04.2016**
(21) Application number: 14797157.6
(22) Date of filing: 13.05.2014
(51) Int. Cl.: G01N 33/574, C12Q 1/68, C12M 1/34, C12M 3/00

(54) **METHOD FOR SCREENING CANCER METASTASIS INHIBITOR USING CULTURE OF CELLS OR SPHEROIDICALLY AGGREGATED CELLS IN WHICH LYSYL-TRNA SYNTHETASE IS REGULATED TO BE EXPRESSED OR UNEXPRESSED**

(30) Priority: 15.05.2013 KR 20130055211
(71) Applicant: Medicinal Bioconvergence Research Center, Yeongtong-gu, Suwon-si Gyeonggi-do 443-270 (KR)
(72) Inventor: LEE, Jung Weon, Seoul 151-015 (KR); KIM, Sunghoon, Seoul 135-859 (KR); NAM, Seo-Hee, Daejeon 300-817 (KR); KIM, Dae Gyu, Seoul 135-859 (KR)
(74) Representative: Pearson, Samuel John
(86) International application number: PCT/KR2014/004276
(87) International publication number: WO 2014/185692

(57) **Abstract**

The present invention relates to a method for scanning a cancer metastasis inhibitor by analyzing the activity of lysyl-tRNA synthetase (KRS) in a cancer cell line cultured in a three-dimensional collagen gel environment, and to a method for monitoring the dissemination of cancer cells from aggregated cancer cells, and the epithelial-mesenchymal transition, migration, invasion, and metastasis of cancer cells. Specifically, it was verified that, in the case where a cell line or a spheroidically aggregated cell line, in which KRS has been regulated to be expressed or unexpressed, was constructed by using various colorectal cancer cells including HCT116 cell line and then cultured in a two-dimensional environment, the incomplete epithelial-to-mesenchymal transition phenotype (incomplete ECM phenotype) was induced in the cell line inhibiting KRS expression, and the inhibition of KRS expression inhibited cell-extracellular matrix (ECM) adhesion and cell-ECM signaling activity. In addition, it was verified that, in the case where the constructed spheroid cell line was cultured in an aqueous environment or a three-dimensional collage gel culture environment, the inhibition of KRS expression induced cells into mesenchymal cells but failed to reach the disintegration of cell-cell adhesion; inhibited the cell-ECM adhesion and the related signaling activity, causing the inhibition of the dissemination of cells from spheroid cells cultured in a three-dimensional collagen gel culture environment; and failed to induce the dissemination of cells through TGFβ1 present in the cellular microenvironment. Thus, the present invention can be used as a method for screening a cancer metastasis inhibitor and a method for monitoring the migration, invasion and metastasis of cancer cells, and will be useful as one of the screening methods capable of creating low-cost, high-efficient added value at the time of pre-clinical tests required for drug development.

Sequence Listing

<110>    Medicinal Bioconvergence Research Center

<120>    Screening method for metastasis inhibitor of cancer using
         spheroid cell mass regulated expression of lysyl-tRNA synthetase
         in 3-dimensional collagen gels environments

<130>    13P-04-43

<160>    12

<170>    KopatentIn 1.71

<210>    1
<211>    58
<212>    DNA
<213>    Artificial Sequence

<220>
<223>    shKRS-0

<400>    1
ccggcctgga agtgacttgc atcaactcga gttgatgcaa gtcacttcca ggtttttg          58

<210>    2
<211>    58
<212>    DNA
<213>    Artificial Sequence

<220>
<223>    shKRS-1

<400>    2
ccggcgtgga cccaaatcaa tactactcga gtagtattga tttgggtcca cgtttttg          58

<210>    3
<211>    58

<212>    DNA
<213>    Artificial Sequence

<220>
<223>    shKRS-2

<400>    3
ccggccagag atacttggac ttgatctcga gatcaagtcc aagtatctct ggtttttg          58

<210>    4
<211>    58
<212>    DNA
<213>    Artificial Sequence

<220>
<223>    shKRS-5

<400>    4
ccgggccttt catcacttat cacaactcga gttgtgataa gtgatgaaag gctttttg          58

<210>    5
<211>    20
<212>    DNA
<213>    Artificial Sequence

<220>
<223>    hEcad-5

<400>    5
tgcccagaaa atgaaaaagg                                                    20

<210>    6
<211>    20
<212>    DNA

<213>    Artificial Sequence

<220>
<223>    hEcad-3

<400>    6
gtgtatgtgg caatgcgttc                                                    20

<210>    7
<211>    20
<212>    DNA
<213>    Artificial Sequence

<220>
<223>    hNcad-5

<400>    7
acagtggcca cctacaaagg                                                    20

<210>    8
<211>    20
<212>    DNA
<213>    Artificial Sequence

<220>
<223>    hNcad-3

<400>    8
ccgagatggg gttgataatg                                                    20

<210>    9
<211>    20
<212>    DNA
<213>    Artificial Sequence

<220>
<223>    hVim-5

<400>    9
gagaactttg ccgttgaagc                                                    20

<210>    10
<211>    20
<212>    DNA
<213>    Artificial Sequence

<220>
<223>    hVim-3

<400>    10
gcttcctgta ggtggcaatc                                                    20

<210>    11
<211>    20
<212>    DNA
<213>    Artificial Sequence

<220>
<223>    hTwist-5

<400>    11
ggagtccgca gtcttacgag                                                    20

<210>    12
<211>    20
<212>    DNA
<213>    Artificial Sequence

<220>
<223>    hTwist-3
<400>    12
tctggaggac ctggtagagg                                                    20

## Description

### BACKGROUND OF THE INVENTION

### 1. Field of the Invention

The present invention relates to a method for screening a cancer metastasis inhibitor by analyzing the cell/cell adhesion in cancer cells, the cell/extracellular matrix (ECM) adhesion, the activation of cell adhesion signaling, and the dissemination of cancer cells, with a cell line or a spheroidically aggregated cell line, in which lysyl-tRNA synthetase has been regulated to be expressed or unexpressed, cultured in a three-dimensional collagen gel environment, and to a method for monitoring the migration, invasion, and metastasis of cancer cells.

### 2. Description of the Related Art

It is known that 90% of death of cancer is attributed to metastasis. Metastasis begins when cancer cells are migrated into a specific organ by blood flow and other factors. The migrated cancer cells grow again in the new place with interaction. Most cancers are developed in epithelial cells forming the wall of organ. Epithelial cells express such proteins involved in cell adhesion and migration as FAK, Src, paxillin, and ERKs. When the activation and expression of such proteins are regulated, various related functions such as cell/cell adhesion, cell/extracellular matrix (ECM) adhesion, migration, and invasion can also be regulated (Petrie and Yamada, 2012; WehrleHaller, 2012). The mentioned proteins are involved in the conversion of epithelial cells into highly motile mesenchymal cells when cell/cell adhesion is disseminated, resulting in the inducement of epithelial-to-mesenchymal transition (EMT) (Bolos et al., 2010). Such EMT not only plays an important role in the development of embryo but also might cause diseases by destroying tissues which are represented by fibrosis and cancer. Fibrosis reduces parenchymal cells responsible for the normal functions and causes the accumulation of collagen, resulting in the loss of tissue functions. The transformed cell morphology, skeletal structure, collagen generation, and migration are suspected to be reasons for fibrosis (Kalluri and Neilson, 2003). Such EMT phenomenon is observed in cancer as well. Highly motile cancer cells are disseminated from aggregated cancer cells by EMT, which are travelling through blood stream or lymphatic system to reach a new destination where they grow to form a tumor again. That is, EMT characterized by the transition of polarized epithelial cells into motile cells plays an important role in the differentiation of carcinoma and the malignant progress thereof (Meng and Wu, 2012). In the meantime, according to the recent reports about circulating tumor cell (CTC), the heterogeneity of metastatic cell, it was verified that the colonization of incomplete EMT phenotype cells maintaining epithelial type like characteristics at the distal metastatic site was advantageous for the metastasis (Thiery and Lim, 2013; Yu et al., 2013).

Various tissue cells maintain their characteristics depending on their unique microenvironments. So, if such microenvironment loses its control over cells, cells would be mal-functioning with displaying degeneration, abnormal differentiation, incontrollable proliferation, etc, resulting in the development of disease like cancer (Sansone and Bromberg, 2011). The reason of difference in metastasis and treatment effect among cancer patients is the effect of microenvironment surrounding a tumor, in addition to cancer cell itself.

Various functions of metastatic cancer cells including cell migration and invasion are affected by the microenvironment surrounding the cells in the course of metastasis. In the microenvironment, various growth factors or cytokines such as TGFβ1 and TNFα are secreted, making optimum environment for tumor cell growth, migration, and invasion. Since the tumor microenvironment can regulate the differentiation and various functions including cell proliferation, survival, migration, and invasion, it is necessary to understand such a microenvironment fully before applying the microenvironment to cancer studies or clinical treatment (Friedl et al., 2012). Anticancer agents nowadays are largely focused on the proliferation of cancer cell itself or the changes of cell signaling, suggesting that cancer development and progress resulted from the changes of microenvironment surrounding tumor cells cannot be regulated with these drugs, that means cancer development and metastasis cannot be controlled fundamentally. Therefore, a novel anticancer strategy to inhibit metastasis can be provided by disclosing the molecular mechanism of cell functions particularly adhesion, EMT, migration, and invasion, via analysis and studies about organic networking between cancer cells and the surrounding cell environment (Friedl et al., 2012).

The *in vitro* cell culture in a two-dimensional environment indicates the cell culture in the conventional plastic flask or on the dish to observe flat cells. To copy the *in vivo* environment, the three-dimensional cell culture has been tried to supplement the disadvantage of the two-dimensional culture displaying artificial cell morphology (Nyga et al., 2011). Cells often display a longish morphology *in vivo* unlike in the *in vitro* two-dimensional environment. So, the *in vitro* three-dimensional cell culture can be a good alternative in cell culture to study the real cell morphology and functions and further the interaction between cells and microenvironment (Aref et al., 2013). It is necessary to verify how cancer metastasis related cell functions including adhesion, EMT, migration, and invasion are regulated by the interaction between cells and the surrounding three-dimensional microenvironment, which would be useful for the development of a metastasis inhibitor.

Aminoacyl-tRNA synthetase combines tRNA with the corresponding amino acid in cells, and the resulting aminoacyl-tRNA is transferred into the elongation factor, ribosome, which would be used for the protein synthesis. Binding specificity of the aminoacyl-tRNA synthetase to the amino acid and tRNA is a very critical factor for maintaining the accuracy of protein synthesis. One of the components forming the aminoacyl-tRNA synthetase, lysyl-tRNA synthetase (KRS) forms a giant molecular complex functioning as a molecular reservoir to regulate various functions of the protein composing aminoacyl-tRNA synthetase in some mammals. Human lysyl-tRNA synthetase contains the unique N-terminal elongation site involved in the interaction between RNA and other proteins and can promote the metastasis function of colorectal cancer cells, suggesting that it plays an important role in metastasis (Kim et al., 2012, FASEB J. 26(10):4142-59).

Therefore, the present inventors analyzed the functions of lysyl-tRNA synthetase (KRS) in the cancer cells cultured in a three-dimensional environment and tried to develop a method for screening a metastasis inhibitor thereby. In the course of such an effort, the inventors prepared a spheroidically aggregated cell line, in which KRS has been regulated to be expressed or unexpressed, by using HCT116, the colorectal cancer cell line. Then, the inventors observed the cell line in the culture condition of extracellular matrix coated two-dimensional environment or three-dimensional cell culture environment surrounded by extracellular matrix or aqueous three-dimensional environment. As a result, it was verified that incomplete epithelial-to-mesenchymal transition phenotype (incomplete ECM phenotype) was induced in the cell line where KRS expression was inhibited, and accordingly the cell-ECM adhesion and the related signaling activity were inhibited. When the spheroidically aggregated cell line was cultured in a three-dimensional culture environment, the inhibition of KRS expression inhibited dissemination but induced mesenchymal cells, but failed to reach disintegration of cell-cell adhesion; inhibited cell-ECM adhesion and its relating ERKs activation; inhibited signaling activity such as paxillin expression and phosphorylation, resulting in the inhibition of dissemination of cells into the microenvironment. The present inventors also verified that such KRS-dependent cancer cell dissemination was consistent with the KRS and ERKs/paxillin distribution/accumulation in the edge of cancer invasion, observed in the stained cancer tissues of colorectal cancer patients, leading to the completion of the present invention.

### SUMMARY OF THE INVENTION

It is an object of the present invention to provide a method for screening a metastasis inhibitor.

It is another object of the present invention to provide a method for monitoring the dissemination of cancer cells from aggregated cancer cells, the epithelial-mesenchymal transition, the migration, invasion, and metastasis of cancer cells, and the expression and activity of the related signaling factors.

To achieve the above objects, the present invention provides a method for screening a metastasis inhibitor comprising the following steps:
1) culturing a cancer cell line or aggregated cancer cells wherein lysyl-tRNA synthetase (KRS) is regulated to be expressed or unexpressed in a three-dimensional environment or in the environment surrounded by extracellular matrix;
2) treating specimens to the cancer cell line or the aggregated cancer cells of step 1);
3) analyzing the activity of KRS in the cancer cell line or the aggregated cancer cells of step 2); and
4) selecting the specimens that have been confirmed to inhibit KRS activity in step 3).

The present invention also provides a method for monitoring the dissemination of cancer cells from aggregated cancer cells, the epithelial-mesenchymal transition, the migration, invasion and metastasis of cancer cells, and the expression and activity of the related signaling factors, comprising the following steps:
1) culturing a cancer cell line or aggregated cancer cells wherein lysyl-tRNA synthetase (KRS) is regulated to be expressed or unexpressed in a three-dimensional environment or in the environment surrounded by extracellular matrix;
2) analyzing the activity of KRS in the cancer cell line or the aggregated cancer cells of step 1); and
3) analyzing the level of metastasis of the cancer cell line or the aggregated cancer cells based on the analyzed KRS activity of step 2).

### ADVANTAGEOUS EFFECT

The present invention can be used as a method for screening a metastasis inhibitor or a method for monitoring the dissemination of cancer cells from aggregated cells, the epithelial-mesenchymal transition, the migration, invasion and metastasis of cancer cells, and the expression and activity of the related signaling factors by analyzing the lysyl-tRNA synthetase (KRS) dependent cell/cell adhesion, cell-extracellular matrix (ECM) adhesion, cell adhesion signaling activity, and cell dissemination in the cancer cell line cultured in a three-dimensional environment or the environment surrounded by extracellular matrix. This invention can also be used as one of the screening methods capable of creating low-cost, high-efficient added value at the time of pre-clinical tests required for drug development.

### BRIEF DESCRIPTION OF THE DRAWINGS

The application of the preferred embodiments of the present invention is best understood with reference to the accompanying drawings, wherein:
Figure 1A is a schematic diagram illustrating the cell culture in a three-dimensional environment, wherein the spheroidically aggregated cells obtained by hanging drop culture are cultured in a 3D environment.
Figure 1B is a diagram illustrating the comparison of the morphology of the cultured spheroidically aggregated cells obtained by hanging drop culture and the cells cultured in a two-dimensional environment.
Figure 1C is a diagram illustrating the increase of the E-cadherin expression over the time in the spheroidically aggregated cells cultured by hanging drop culture method.
Figure 1D is a diagram illustrating the morphology of the spheroidically aggregated cells cultured in a 3D collagen gel environment, observed under optical microscope.
Figure 1E is a diagram illustrating the expression of KRS in the spheroidically aggregated cells obtained by hanging drop culture with the cell lines transfected with shRNA inhibiting lysyl-tRNA synthetase (KRS).
   Mock: control, and
   0-3, 2-1, 2-2, 5-3: cell line clones in which KRS expression has been suppressed.
Figure 1F is a diagram illustrating the expression of epithelial-to-mesenchymal transition (EMT) related marker mRNA in the spheroidically aggregated cells cultured by hanging drop culture.
   P: HCT116 parental cells, control,
   2-1, 2-2, 2-3, 5-4: cell line clones in which KRS expression has been suppressed, and
   KRS WT: KRS over-expressing cell line.
Figure 1G is a diagram illustrating the expression of EMT related marker protein in the spheroidically aggregated cells cultured by hanging drop culture.
   Mock: control, and
   shKRS: cell line clone in which KRS expression has been suppressed.
Figure 2A is a diagram illustrating the expression of KRS dependent EMT marker protein in the cells cultured normally in a two-dimensional (2D) cell culture condition supplemented with serum.
   P: control,
   2-1, 2-2, 2-3, 5-4: cell line clones in which KRS expression has been suppressed, and
   WT: KRS over-expressing cell line.
Figure 2B is a diagram illustrating the expression of EMT related marker protein in the cells cultured normally in a two-dimensional (2D) cell culture condition supplemented with 10% serum which was treated with nothing or treated with TNFα or TGFβ1.
   Parental: control,
   shKRS 2-1: cell line clone 2-1 in which KRS expression has been suppressed, and
   KRS WT: KRS over-expressing cell line.
Figure 2C is a diagram illustrating the inhibition of E-cadherin and β-catenin expressions by regulating KRS expression in the cells cultured normally in a two-dimensional environment supplemented with 10% serum.
   Parental: control,
   shKRS: cell line clone in which KRS expression has been suppressed, and
   KRS WT: KRS over-expressing cell line.
Figure 2D is a diagram illustrating the morphology of cells growing in colony as being attached on the culture plate in a normal two-dimensional (2D) environment supplemented with 10% serum, observed under phase contrast microscope.
   KRS-neg: cell line in which KRS expression has been suppressed, and
   KRS-pos: HCT116 parental cells or KRS over-expressing cell line.
Figure 3 is a diagram illustrating the result of cell culture on the laminin coated dish or cover-glass in a two-dimensional environment with 2% serum which was reduced from the normal concentration in order to minimize the effect of growth factors in the serum,
Figure 3A is a diagram illustrating that the cell-adhesion mediated phosphorylations of Src, ERKs, and paxillin were reduced significantly but the phosphorylation of FAK was not change in the cell lines (shKRS₂₋₁, shKRS₅₋₄) in which KRS expression was suppressed,
Figure 3B is a diagram illustrating that the phosphorylation level of ERKs was high in the parental cells expressing KRS and in the cell line over-expressing KRS but the phosphorylation level of ERKs was significantly low in those cell lines where KRS expression was suppressed,
Figure 3C is a diagram illustrating the high phosphorylation level of paxillin in the parental cell line expressing KRS or in the cell line over-expressing KRS and the significantly low phosphorylation level of paxillin in the cell line where KRS expression was suppressed,
Figure 3D is a diagram illustrating that the cell morphology or spreading was not much different according to the suppression of KRS in HCT116 cells,
Figure 3E is a diagram illustrating the result of immunofluorescence, wherein the phosphorylation level of ERKs was high in the parental cell line expressing KRS or in the cell line over-expressing KRS, but the phosphorylation level of ERKs decreased significantly when the cell line was treated with YH16899 (Kim DG et al., Nat Chem Biol. 2014 Jan; 10(1):29-34), the KRS inhibitor,
Figure 3F is a diagram illustrating the result of immunofluorescence, wherein the phosphorylation level of Tyr118 was high in the parental cell line expressing KRS or in the cell line over-expressing KRS, but the phosphorylation level was significantly reduced when the cell line was treated with YH16899 (Kim DG et al., Nat Chem Biol. 2014 Jan; 10(1):29-34), the KRS inhibitor,
Figure 3G is a diagram illustrating that there was no big difference in cell morphology or spreading in relation to the phosphorylation of FAK Tyr397 in the parental cells expressing KRS or in the cell line over-expressing KRS treated with YH16899.
Figure 4A is a diagram illustrating the inhibition of FAK and ERKs phosphorylations according to the regulation of KRS expression in the cells cultured in a normal two-dimensional environment supplemented with 10% serum.
   P: control,
   2-1, 5-4: cell line clones in which KRS expression has been suppressed, and
   myc-KRS WT: myc-KRS over-expressing cell line.
Figure 4B is a diagram illustrating the inhibition of s-Src and paxillin phosphorylations according to the regulation of KRS expression in the cells cultured in a normal two-dimensional environment supplemented with 10% serum.
   P: control, and
   2-1, 5-4: cell line clones in which KRS expression has been suppressed.
Figure 4C is a diagram illustrating the phosphorylations of FAK and ERKs in the cells cultured in the fibronectin coated culture dish in suspension or in a two-dimensional environment for 2 hours, wherein the expression of KRS was regulated.
   S: suspension,
   FN: cultured in the fibronectin coated culture dish,
   P: control,
   2-1, 5-4: cell line clones in which KRS expression has been suppressed, and
   myc-KRS WT: KRS over-expressing cell line.
Figure 4D is a diagram illustrating the inhibition of focal adhesion, confirmed by observing tyrosine 397 phosphorylated FAK in the cell line, in which KRS expression was suppressed, cultured in the fibronectin coated culture dish in a two-dimensional environment for 2 hours.
   P: control,
   2-1, 5-4: cell line clones in which KRS expression has been suppressed, and
   myc-KRS WT: myc-KRS over-expressing cell line.
Figure 5 is a diagram illustrating the changes of extracellular matrix (ECM) adhesion related signaling activity observed when the spheroidically aggregated cell line, in which KRS has been regulated to be expressed or unexpressed, was cultured in a three-dimensional collagen gel environment for 3 ∼ 24 hours.
   Parental: control,
   2-1, 2-2, 2-3, 5-4: cell line clones in which KRS expression has been suppressed, and
   KRS WT: KRS over-expressing cell line.
Figure 6A is a diagram illustrating the phosphorylation of ERKs observed under confocal microscope after performing immunostaining of p-ERKs in HCT116 cells expressing KRS normally and in the spheroidically aggregated cell lines, in which KRS expression was suppressed (shKRS₂₋₁, shKRS₅₋₄), cultured in a three-dimensional collagen gel environment.
Figure 6B is a diagram illustrating paxillin observed under confocal microscope after performing immunostaining of paxillin in HCT116 cells expressing KRS normally and in the spheroidically aggregated cell line, in which KRS expression was suppressed (shKRS₂₋₁), cultured in a three-dimensional collagen gel environment.
Figure 6C is a diagram illustrating the phosphorylation level of ERKs observed under confocal microscope after performing immunostaining of p-ERKs in the spheroidically aggregated cell line, in which KRS was normally expressed (HCT116), treated with the control DMSO (dimethyl sulfoxide) or YH16899, the KRS inhibitor, and cultured in a three-dimensional collagen gel environment.
Figure 6D is a diagram illustrating the expression level of paxillin observed under confocal microscope after performing immunostaining of paxillin in the spheroidically aggregated cell line, in which KRS was normally expressed (HCT116), treated with the control DMSO (dimethyl sulfoxide) or U0126, the selective inhibitor of MEK/ERK kinase, and cultured in a three-dimensional collagen gel environment.
Figure 7A is a diagram illustrating the changes of cell-ECM adhesion related signaling activity according to the treatment of ERKs inhibitor to the spheroidically aggregated HCT116 parental cells cultured in a three-dimensional collagen gel environment.
   U0126: ERKs inhibitor.
Figure 7B is a diagram illustrating the changes of cell-cell adhesion related gene mRNA expression according to the treatment of ERKs inhibitor to the spheroidically aggregated HCT116 parental cells cultured in a three-dimensional collagen gel environment.
   U0126: ERKs inhibitor.
Figure 8A is a diagram illustrating the changes of EMT related protein in the spheroidically aggregated cells cultured in a three-dimensional collagen gel environment.
   P: control,
   2-1, 2-2: cell line clones in which KRS expression has been suppressed, and
   KRS WT: KRS over-expressing cell line.
Figure 8B is a diagram illustrating the changes of snail1 expression in the spheroidically aggregated cells cultured in a three-dimensional collagen gel environment for 5 days.
   Parental: control, and
   shKRS₂₋₁, shKRS₂₋₂: cell line clones in which KRS expression has been suppressed.
Figure 8C is a diagram illustrating the inhibition of E-cadherin expression in the spheroidically aggregated cells cultured in a three-dimensional collagen gel environment for 3 days.
   Parental: control,
   shKRS: cell line clones in which KRS expression has been suppressed, and
   KRS WT: KRS over-expressing cell line.
Figure 9A is a diagram illustrating that the expression of CK14 (epithelial marker cytokine 14) is high in the parental cells expressing KRS or in the cell line over-expressing KRS cultured in a three-dimensional collagen gel environment and is normally induced in those cells disseminated from the edge of spheroid (white arrow head), while the expression of CK14 is low in the cells wherein KRS was suppressed and in those cells disseminated from the spheroid.
Figure 9B is a diagram illustrating that the expression of CK14 (epithelial marker cytokine 14) is high in the parental cells expressing KRS or in the cell line over-expressing KRS cultured in a three-dimensional collagen gel environment and is normally induced in those cells disseminated from the edge of spheroid (white arrow head), while the expression of CK14 is low in general when the cells, even though they express KRS, are treated with the MEK/ERK inhibitor U1026 or the KRS inhibitor YH16899 or in the cells disseminated from the spheroid.
Figure 10A is a diagram illustrating that the dissemination of cells from the spheroidically aggregated cells cultured in a three-dimensional collagen gel environment was not observed when KRS expression was inhibited.
   Parental: control,
   2-1, 2-2, 2-3, 5-4: cell line clones in which KRS expression has been suppressed, and
   KRSWT: KRS over-expressing cell line.
Figure 10B is a diagram illustrating the expression level of TGFβ1 protein in the spheroidically aggregated cells cultured in a three-dimensional collagen gel environment.
   Parental: control,
   2-1, 2-2, 2-3, 5-4: cell line clones in which KRS expression has been suppressed, and
   KRSWT: KRS over-expressing cell line.
Figure 11A is a diagram illustrating the cell dissemination from the spheroidically aggregated cells cultured in a three-dimensional collagen gel environment according to the treatment of TNFα.
   Parental: control,
   shKRS: cell line in which KRS expression has been suppressed, and
   KRS WT: KRS over-expressing cell line.
Figure 11B is a diagram illustrating the cell dissemination from the spheroidically aggregated cells cultured in a three-dimensional collagen gel environment according to the treatment of TGFβ1.
   Parental: control,
   shKRS: cell line in which KRS expression has been suppressed, and
   KRS WT: KRS over-expressing cell line.
Figure 12 is a diagram illustrating that the dissemination of cells from the spheroidically aggregated cells cultured in a three-dimensional collagen gel environment was inhibited when the aggregated cells were treated with TGFβ and ERKs inhibitors.
   U0126: ERKs inhibitor.
Figure 13A is a diagram illustrating the measurement of ERKs activity in HCT116 parental cells, the cell line where KRS expression was inhibited, and the cell line over-expressing KRS, by using FRET technique after transfecting those cells with EKAR (Fluorescence Resonance Energy Transfer (FRET)-based ERKs activity indicators, Harvey et al., 2008 PNAS 105:19264-19269). High ERKs phosphorylation was presented by red color and low phosphorylation was presented by blue color.
Figure 13B is a diagram illustrating the statistic results of ERK activity measured by FRET technique with HCT116 parental cells (parental, P), the cell lines where KRS expression was inhibited [shKRS₂₋₁ and shKRS₅₋₄] and the cell line over-expressing KRS, which were respectively transfected with EKAR.
Figure 14 is a diagram illustrating the paxillin expression and Tyr118 phosphorylation in HCT116 parental cells (parental, P), the cell lines where KRS expression was inhibited [shKRS₂₋₁ and shKRS₅₋₄], and the cell line over-expressing KRS (KRS WT), which were respectively transfected temporarily with pCMV-Mock (M), pCMV-paxillin(paxillin) (Px), and pCMV-ERK1/2 (Ek).
Figure 15A is a diagram illustrating the binding activity of integrin α6, integrin β1, KRS, and p67LR (p67 laminin receptor) and any changes according to the treatment of YH16899 (YH) investigated after performing coimmunoprecipitation of HCT116 cell line over-expressing myc-KRS in a normal two-dimensional condition in the presence of 10% serum.
Figure 15B is a diagram illustrating the binding activity of integrin α6, integrin β1, KRS, and p67LR (p67 laminin receptor) and any changes according to the treatment of YH16899 (YH) investigated after performing coimmunoprecipitation of HCT116 cell line over-expressing myc-KRS in the laminin coated (10 mg/ml) plate in a normal two-dimensional condition in the presence of 2% serum.
Figure 16A is a diagram illustrating that KRS dependently activated ERKs related to c-Jun phosphorylation rather than Elk-1, known as a downstream transcription factor, investigated by using the spheroidically aggregated cells cultured in a three-dimensional collagen gel environment.
Figure 16B is a diagram illustrating the binding sites of c-Jun and Elk-1 located in the paxillin promoter region and the non-binding sites thereof for the comparison.
Figure 16C is a diagram illustrating the result of ChIP (chromatin immunoprecipitation) analysis with HCT116 parental cells cultured as spheroid in a three-dimensional collagen gel environment treated with DMSO (control) or YH16899, and shKRS₂₋₁ wherein KRS expression was suppressed by using c-Jun antibody. In this diagram, c-Jun binding site is shown in the control.
Figure 16D is a diagram illustrating the result of ChIP analysis with HCT116 parental cells cultured as spheroid in a three-dimensional collagen gel environment treated with DMSO (control) or U1026 or YH16899, and shKRS₂₋₁ wherein KRS expression was suppressed by using Elk-1 antibody.
Figure 17A is a diagram illustrating the inhibition of the dissemination of cells from the spheroid in a three-dimensional collagen gel according to the inhibition of KRS expression in the colorectal cancer cell line SW620.
Figure 17B is a diagram illustrating the decrease of the phosphorylation of ERKs and the decrease of both paxillin expression and phosphorylation in the colorectal cancer cell line SW620 treated with the KRS inhibitor YH16899.
Figure 18A is a diagram illustrating that the expression and phosphorylation of the focal adhesion related molecule such as paxillin were reduced and the expression of the epithelial marker was increased in HCT116 cell line in a three-dimensional collagen gel when the cell line was treated with the ERK inhibitor U0126.
Figure 18B is a diagram illustrating that the expression and phosphorylation of the focal adhesion related molecule such as paxillin were reduced and the expression of the epithelial marker was also decreased, in addition to the phosphorylation of ERKs, in HCT116 cell line in a three-dimensional collagen gel when the cell line was treated with the KRS inhibitor YGH16899.
Figure 19A is a diagram illustrating the result of investigation of signaling, wherein the cells pre-treated with integrin α6 inhibiting antibody were cultured in the laminin coated 2D environment for 2 hours and then ERKs activity was measured. As a result, ERKs activity was reduced, suggesting that FAK and ERKs did not interact in signaling.
Figure 19B is a diagram illustrating the result of investigation of FAK phosphorylation and ERKs activity, wherein HCT116 parental cells were infected with Ad-HA control adenovirus or dead form adenovirus wherein Ad-HA-R454 FAK kinase activity was eliminated, or the cell line in which KRS expression was suppressed was infected with Ad-HA control virus, Ad-HA ΔN(1-100) FAK, or Ad-HA-FAK WT virus, followed by the investigation of FAK phosphorylation and ERKs activity. As a result, it was confirmed that FAK and ERKs activity did not related to each other.

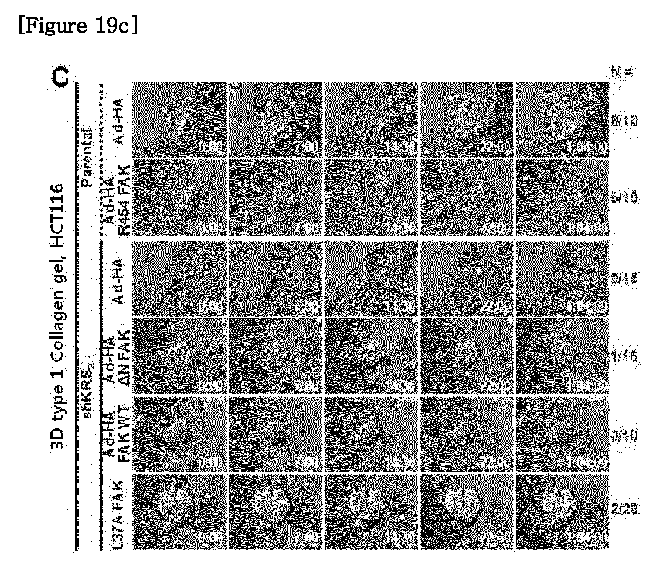

Figure 19C is a diagram illustrating the result of the observation of cell dissemination by using time-lapse microscope, wherein HCT116 parental cells were infected with Ad-HA-control virus or dead form adenovirus wherein Ad-HA-R454 FAK kinase activity was eliminated, or the cell line in which KRS expression was suppressed (shKRS₂₋₁) was infected with Ad-HA control virus, Ad-HA ΔN(1-100) FAK, or Ad-HA-FAK WT virus, followed by preparation of spheroid of each cell line above. The spheroid of each cell line in a three-dimensional collagen gel was observed under time-lapse microscope and as a result the dissemination of cells from the spheroid was not induced in the cell line wherein KRS expression was suppressed even when FAK was over-expressed therein.

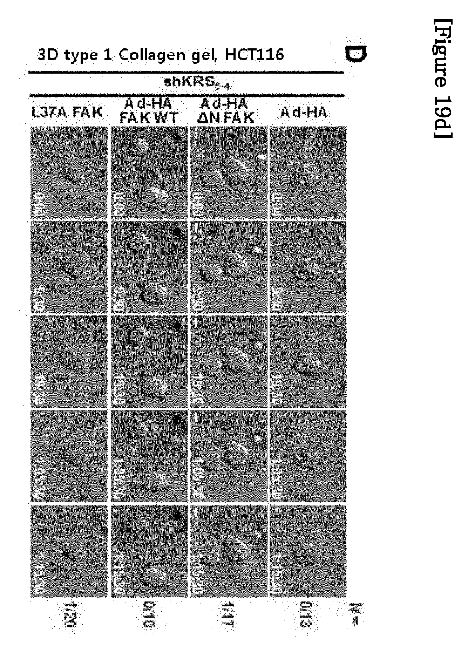

Figure 19D is a diagram illustrating the result of the observation of cell dissemination by using time-lapse microscope, wherein HCT116 parental cells were infected with Ad-HA-control virus or dead form adenovirus wherein Ad-HA-R454 FAK kinase activity was eliminated, or the cell line in which KRS expression was suppressed (shKRS₅₋₄) was infected with Ad-HA control virus, Ad-HA ΔN(1-100) FAK, or Ad-HA-FAK WT virus, followed by preparation of spheroid of each cell line above. The spheroid of each cell line in a three-dimensional collagen gel was observed under time-lapse microscope and as a result the dissemination of cells from the spheroid was not induced in the cell line wherein KRS expression was suppressed even when FAK was over-expressed therein.
Figure 19E is a diagram illustrating that the ERKs activity was not affected by FAK activity in the cell line wherein the active mutant form L37A FAK was expressed.
Figure 20A is a diagram illustrating the expressions of relevant factors in the stable cell line established by transfecting the cell line, in which KRS expression was suppressed, with pCMV-Mock, pCMV-paxillin, or pCMV-ERK1/2.
Figure 20B is a diagram illustrating the result of 28-hour observation of the dissemination of cells using time-lapse microscope to investigate the dissemination of cells from the spheroid in a three-dimensional collagen gel environment which was obtained from the stable cell line constructed by transfecting the cell line, in which KRS expression was suppressed, with pCMV-Mock, pCMV-paxillin, or pCMV-ERK1/2.
Figure 21A is a diagram illustrating the correlation of the expressions of paxillin, p-ERKs, E-cadherin, and KRS in human colon tumor tissues including clinically diagnosed colorectal cancer tissues, investigated by Western blotting.
Figure 21B is a diagram illustrating the correlation of such proteins as paxillin, p-ERKs, and KRS in human colon tumor tissues including those tissues clinically diagnosed as Grade II and III, investigated by immunohistochemical staining.

### DESCRIPTION OF THE PREFERRED EMBODIMENTS

Hereinafter, the present invention is described in detail.

The present invention provides a method for screening a metastasis inhibitor comprising the following steps:
1) culturing a cancer cell line or aggregated cancer cells wherein lysyl-tRNA synthetase (KRS) is regulated to be expressed or unexpressed in a three-dimensional environment or in the environment surrounded by extracellular matrix;
2) treating specimens to the cancer cell line or the aggregated cancer cells of step 1);
3) analyzing the activity of KRS in the cancer cell line or the aggregated cancer cells of step 2); and
4) selecting specimens that have been confirmed to inhibit KRS activity in step 3).

In step 1), the said three-dimensional environment is an aqueous environment or a collagen surrounding environment, but not always limited thereto, and type 1 collagen, laminin, fibronectin, or natural hydrogel such as matrigel or hyaluronic acid, known to those in the art, can be used. It is also possible to combine them at different ratios. The concentration of the said collagen is preferably 1 ∼ 5 mg/mℓ, more preferably 2 ∼ 4 mg/mℓ, and most preferably 2 ∼ 2.5 mg/mℓ. The said collagen is preferably prepared as a neutral, but not always limited thereto. The cancer cell line cultured in the three-dimensional environment indicates the spheroidically aggregated cell line surrounded by extracellular matrix cultured by hanging drop culture in an aqueous solution, but not always limited thereto.

The cancer in step 1) is preferably a metastatic cancer or a metastasis inducible cancer, which is preferably selected from the group consisting of breast cancer, liver cancer, stomach cancer, colon cancer, bone cancer, lung cancer, pancreatic cancer, head/neck cancer, uterine cancer, ovarian cancer, rectal cancer, esophageal cancer, small bowel neoplasm, anal cancer, colon carcinoma, fallopian tube carcinoma, endometrial carcinoma, uterine cervical carcinoma, vaginal carcinoma, vulva carcinoma, Hodgkin's disease, prostatic cancer, bladder cancer, kidney cancer, ureter cancer, renal cell carcinoma, renal pelvic cancer, and central nervous system tumor. In a preferred embodiment of the present invention, colorectal cancer was preferably selected, but not always limited thereto.

The specimen in step 2) is preferably selected from the group consisting of peptide, protein, antibody, antibody fragment, non-peptide compound, active compound, fermented product, cell extract, plant extract, animal tissue extract, and blood plasma, but not always limited thereto.

The KRS activity in step 3) indicates the activity in cancer cells preferably selected from the group consistiing of cell-cell adhesion of cancer cells, cell-extracellular matrix (ECM) adhesion, cell scattering, wound-healing, changing the activity of cell adhesion signaling factors, c-Jun/paxillin promoter binding, and the dissemination of cells from spheroid cells, but not always limited thereto. Herein, the cell-cell adhesion preferably induces the changes in expression or the location of expression of the epithelial marker such as ZO-1, occuludin, CK14, β-catenin or E-cadherin, or the mesenchymal marker such as fibronectin, twist, vimentin, α-SMA (smooth muscle actin), snail-1, Slug or N-cadherin. Also, the said cell-extracellular matrix adhesion preferably induces the changes in the strength of cell-extracellular adhesion, cell spreading, focal adhesion and its failure, actin-reconstruction, and the expression or the location of expression of phosphorylated FAK, c-Src, EKRs, c-Jun or paxillin. The cell adhesion signaling activity induces the decrease or changes in the expression or phosphorylation of FAK, c-Src, ERKs, c-Jun, or paxillin, or the changes of integrin α6 conjugation, but not always limited thereto.

To analyze the KRS activity in step 3), a method can be selected from the group consisting of Western blotting, real-time PCR, co-immunoprecipitation, ChIP (chromatin immunoprecipitation), FRET, immunofluorescence, and immunohistochemistry, according to a preferred embodiment of the present invention, but not always limited thereto, and any method well known to those in the art can be used.

The present invention also provides a method for monitoring the dissemination of cancer cells from cancer cell line or aggregated cancer cells, the epithelial-mesenchymal transition, the migration, invasion and metastasis of cancer cells, and the expression or activity of the related signaling factors, comprising the following steps:
1) culturing a cancer cell line or aggregated cancer cells wherein lysyl-tRNA synthetase (KRS) is regulated to be expressed or unexpressed in a three-dimensional environment or in the environment surrounded by extracellular matrix;
2) analyzing the activity of KRS in the cancer cell line or the aggregated cancer cells of step 1); and
3) analyzing the level of metastasis of the cancer cell line or the aggregated cancer cells based on the analyzed KRS activity of step 2).

In step 1), the said three-dimensional environment is an aqueous environment or a collagen surrounding environment, but not always limited thereto, and type 1 collagen, laminin, fibronectin, or natural hydrogel such as matrigel or hyaluronic acid, known to those in the art, can be used. It is also possible to combine them at different ratios. The concentration of the said collagen is preferably 1 ∼ 5 mg/mℓ, more preferably 2 ∼ 4 mg/mℓ, and most preferably 2 ∼ 2.5 mg/mℓ. The said collagen is preferably prepared as a neutral, but not always limited thereto. The cancer cell line cultured in the three-dimensional environment indicates the spheroidically aggregated cell line surrounded by extracellular matrix cultured by hanging drop culture in an aqueous solution, but not always limited thereto.

The cancer in step 1) is preferably a metastatic cancer or a metastasis inducible cancer, which is preferably selected from the group consisting of breast cancer, liver cancer, stomach cancer, colon cancer, bone cancer, lung cancer, pancreatic cancer, head/neck cancer, uterine cancer, ovarian cancer, rectal cancer, esophageal cancer, small bowel neoplasm, anal cancer, colon carcinoma, fallopian tube carcinoma, endometrial carcinoma, uterine cervical carcinoma, vaginal carcinoma, vulva carcinoma, Hodgkin's disease, prostatic cancer, bladder cancer, kidney cancer, ureter cancer, renal cell carcinoma, renal pelvic cancer, and central nervous system tumor. In a preferred embodiment of the present invention, colorectal cancer was preferably selected, but not always limited thereto.

The KRS activity in step 2) indicates the activity in cancer cells preferably selected from the group consistiing of cell-cell adhesion of cancer cells, cell-extracellular matrix (ECM) adhesion, cell scattering, wound-healing, changing the activity of cell adhesion signaling factors, c-Jun/paxillin promoter binding, and the dissemination of cells from spheroid cells, but not always limited thereto. Herein, the cell-cell adhesion preferably induces the changes in expression or the location of expression of the epithelial marker such as ZO-1, occuludin, CK14, β-catenin or E-cadherin, or the mesenchymal marker such as fibronectin, twist, vimentin, α-SMA (smooth muscle actin), snail-1, Slug or N-cadherin. Also, the said cell-extracellular matrix adhesion preferably induces the changes in the strength of cell-extracellular adhesion, cell spreading, focal adhesion and its failure, actin-reconstruction, and the expression or the location of expression of phosphorylated FAK, c-Src, EKRs, c-Jun or paxillin. The cell adhesion signaling activity induces the decrease or changes in the expression or phosphorylation of FAK, c-Src, ERKs, c-Jun, or paxillin, or the changes of integrin α6 conjugation, but not always limited thereto.

To analyze the KRS activity in step 2), a method can be selected from the group consisting of Western blotting, real-time PCR, co-immunoprecipitation, ChIP (chromatin immunoprecipitation), FRET, immunofluorescence, and immunohistochemistry, according to a preferred embodiment of the present invention, but not always limited thereto, and any method well known to those in the art can be used.

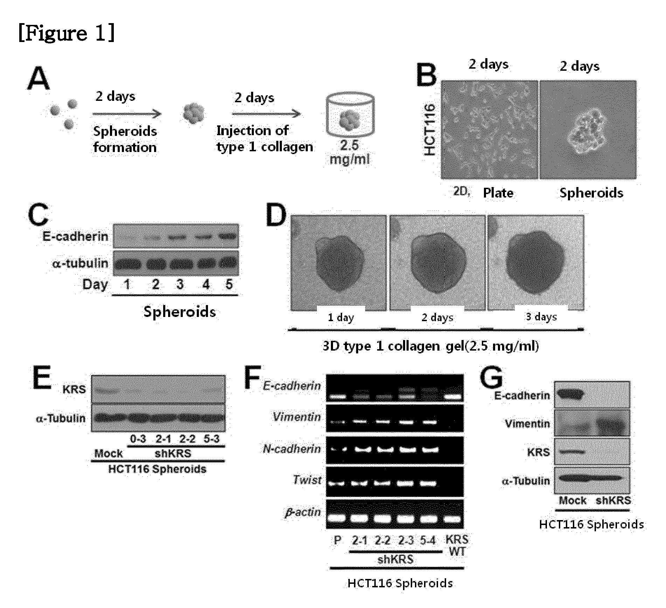

In a preferred embodiment of the present invention, it was confirmed that the expression of E-cadherin in the spheroidically aggregated cells cultured in a two-dimensional environment (see Figure 1B) was increased over the culture time (see Figure 1C). To confirm the effect of lysyl-tRNA synthetase (KRS), the KRS knock-out or over-expressing cell line was constructed and tested (see Figure 1E). The epithelial-to-mesenchymal transition (ETM) marker gene mRNA expression in the constructed KRS knock-out cell line cultured in a three-dimensional environment was also investigated. As a result, the epithelial marker E-cadherin expression was reduced therein, but the mesenchymal markers Vimentin, N-cadherin, and Twist mRNAs were significantly up-regulated (see Figure 1E).

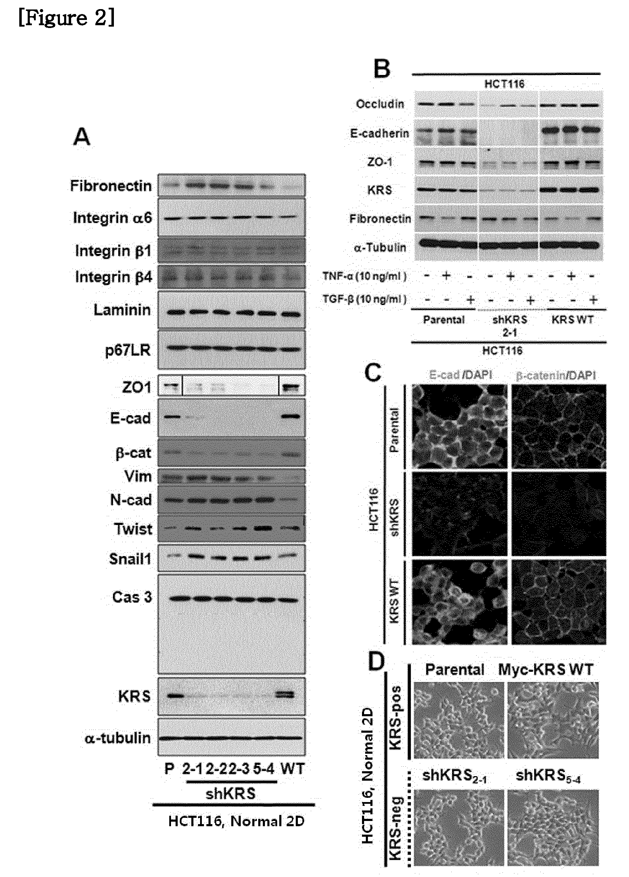

The expression of the epithelial marker in HCT116 parental cells and the cell line over-expressing KRS cultured in a normal two-dimensional environment supplemented with 10% serum was higher than the expression of the mesenchymal marker, while the expression of the epithelial marker in the cell lines wherein KRS expression was suppressed (2-1, 2-1, 2-3, and 5-4) was lower than the expression of the mesenchymal marker (see Figure 2A).

To investigate the effect of the surrounding micro-environmental factors in the cell line wherein KRS expression was suppressed, the cell line was treated with TNFα or TGFβ1. As a result, it was confirmed that the expression of the epithelial marker protein occludin was increased, while the expression of the mesenchymal marker fibronectin was reduced (see Figure 2B).

In the cell line wherein KRS expression was suppressed, the expressions of E-cadherin and β-catenin were inhibited (see Figure 2C), but there was no significant change in cell/cell adhesion (see Figure 2D). Therefore, it was confirmed that the inhibition of the KRS expression caused the inhibition of the epithelial marker expression, the increase of the mesenchymal marker expression, the colony formation and growing without being scattered (see Figures 2C and 2D), and the induction of partial epithelial-mesenchymal transition (EMT).

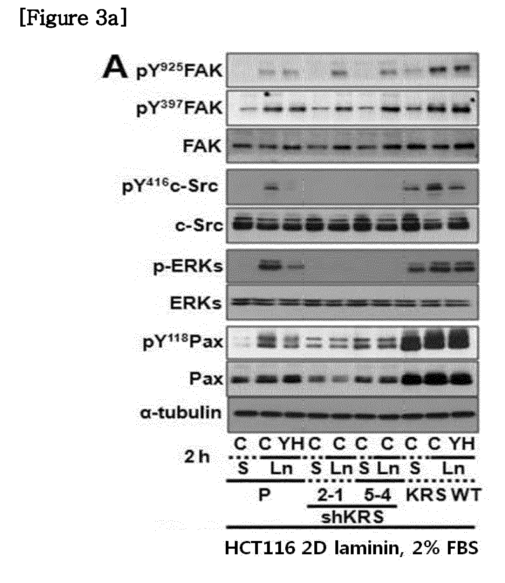

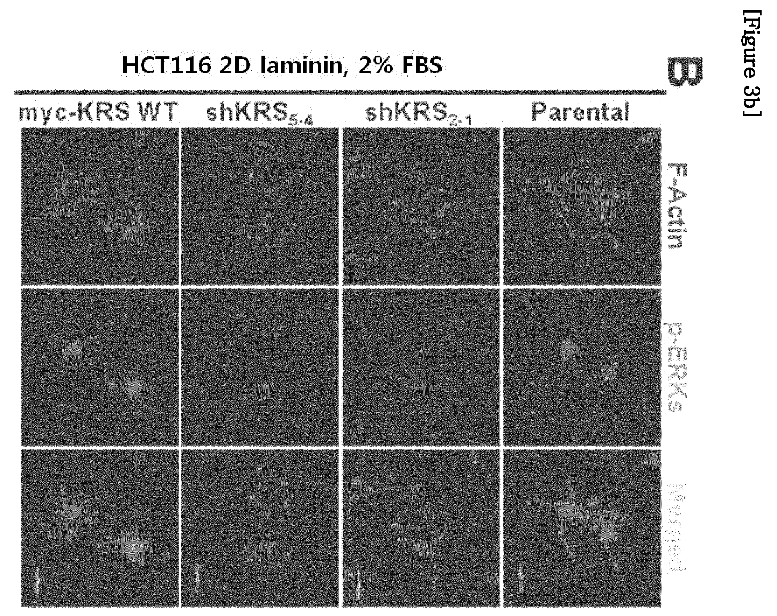

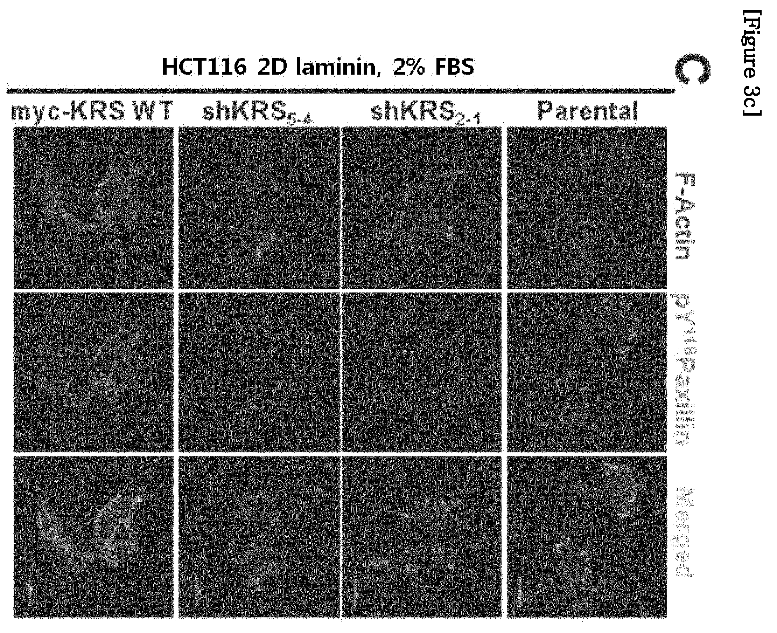

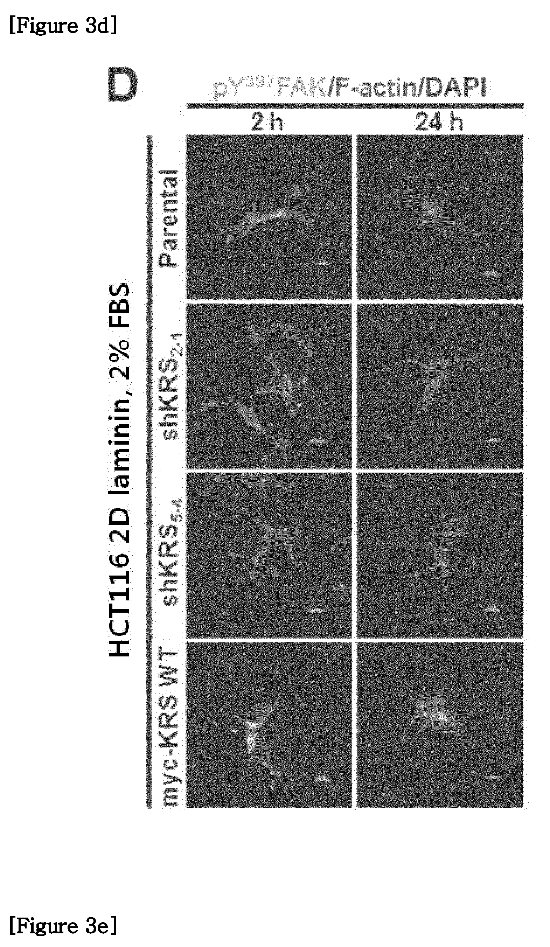

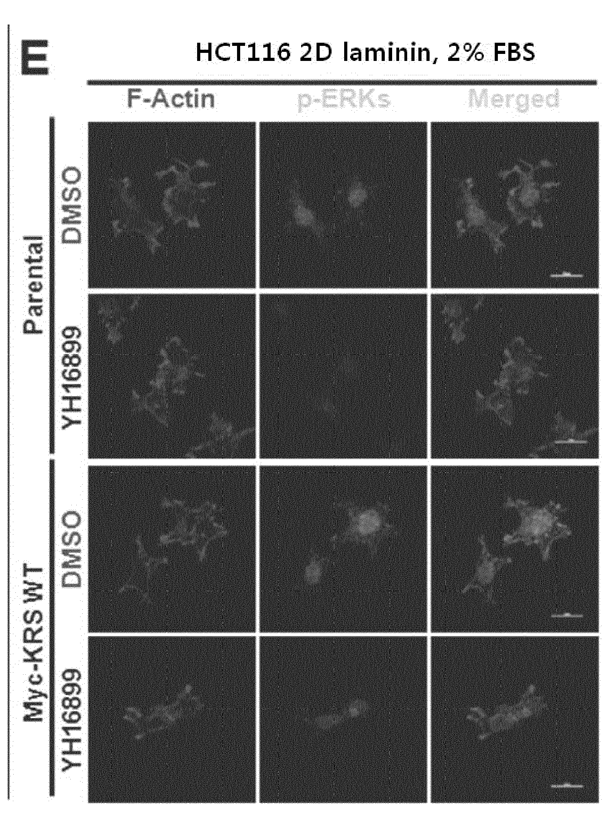

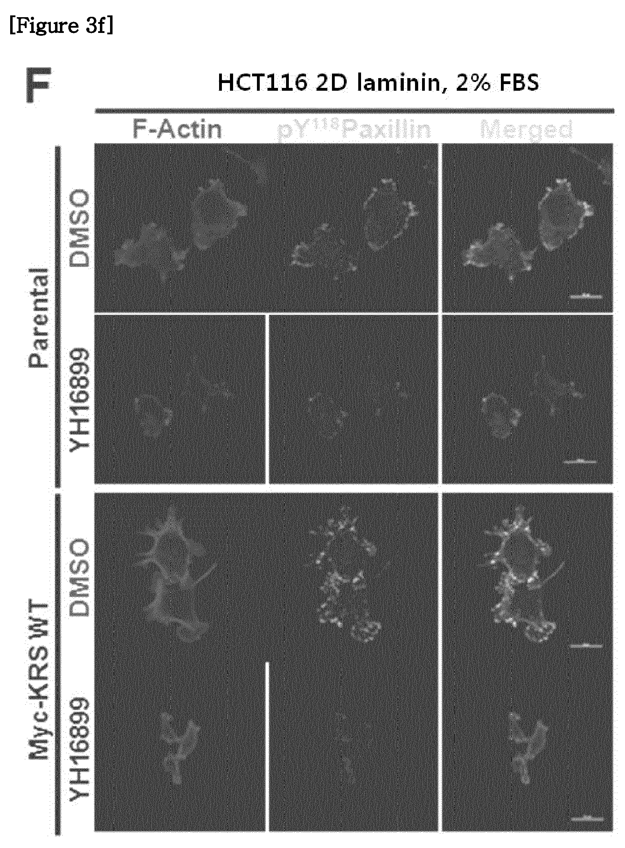

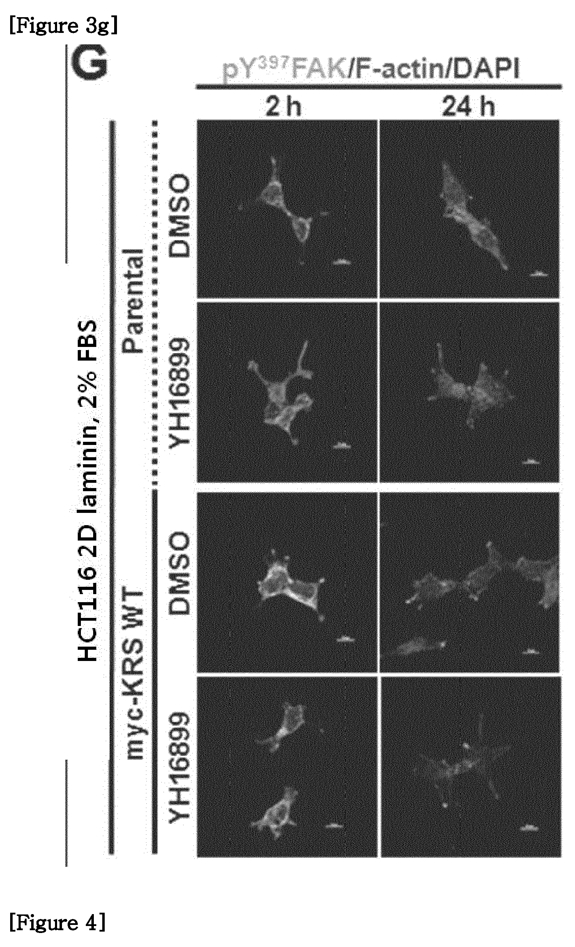

After replating HCT116 parental cells, the cell lines wherein KRS expression was suppressed (shKRS₂₋₁, shKRS₅₋₄), and the cell line over-expressing KRS (Myc-KRS-WT) in the laminin pre-coated culture dish and cover glass, the cells were treated with YH16899 (see Figures 3A, 3E, 3F, and 3G) or not treated (see Figures 3A, 3B, 3C, and 3D), followed by examination. As a result, it was verified that the KRS expression was related not to the FAK phosphorylation but to the expressions and phosphorylations of pERKs and paxillin (see Figure 3A), and accordingly pERKs (see Figures 3B and 3E) and Tyr118 affected focal adhesion happening on the location of phosphorylated paxillin (see Figures 3C and 3F) but did not change the phosphorylation and cell morphology of FAK Tyr397 (see Figures 3D and 3G).

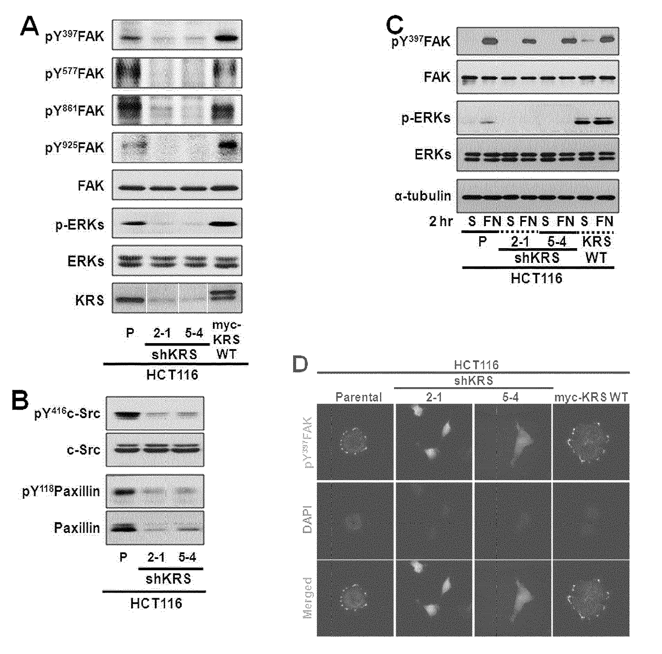

The activity of cell-extracellular matrix (ECM) adhesion related signaling in the cell line wherein KRS expression was suppressed was investigated. As a result, the activities of FAK, ERKs, c-Src, and paxillin were significantly reduced (see Figures 4A and 4B), and thereby the formation of focal adhesion was suppressed (see Figure 4D).

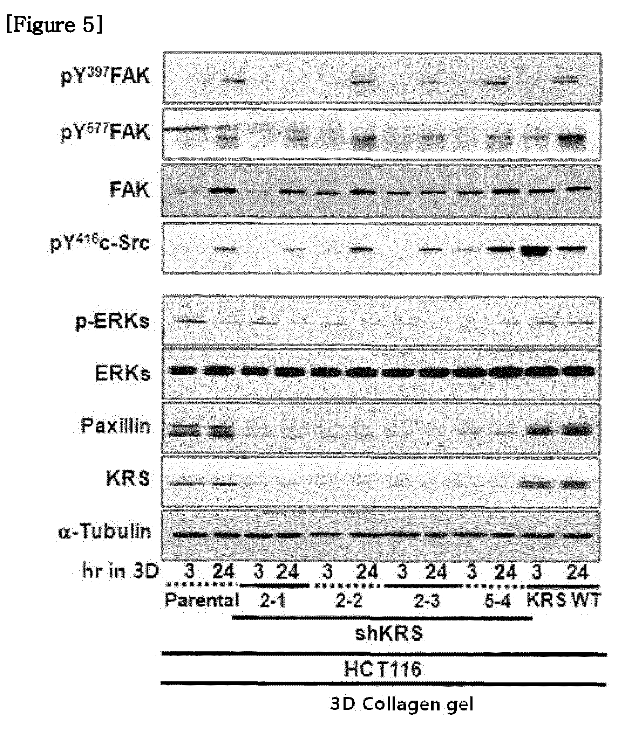

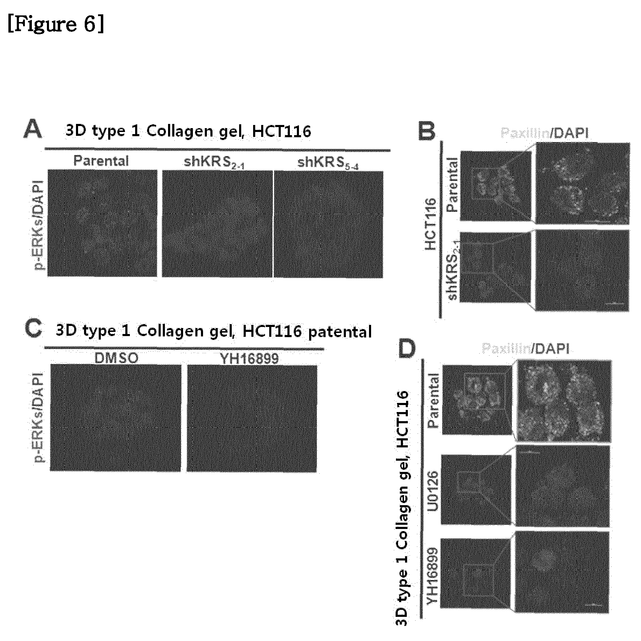

It was also confirmed that the expressions and activities of ERK and paxillin were inhibited in the spheroidically aggregated cell line (see Figure 1D), in which KRS expression was suppressed, cultured in a three-dimensional collagen gel environment (see Figure 5). HCT116 parental cells, KRS knock-out cell lines (shKRS₂₋₁, shKRS₅₋₄), and HCT parental cells treated with U0126 and YH16899 were cultured in a three-dimensional collagen gel for a day, followed by immunostaining to investigate the phosphorylation of ERKs and the expression of paxillin. As a result, it was confirmed that the phosphorylation of ERKs (Figures 6A and 6C) and the expression of paxillin (Figures 6B and 6D) were inhibited significantly in the KRS knock-out cell lines or the cell line treated with KRS inhibitors.

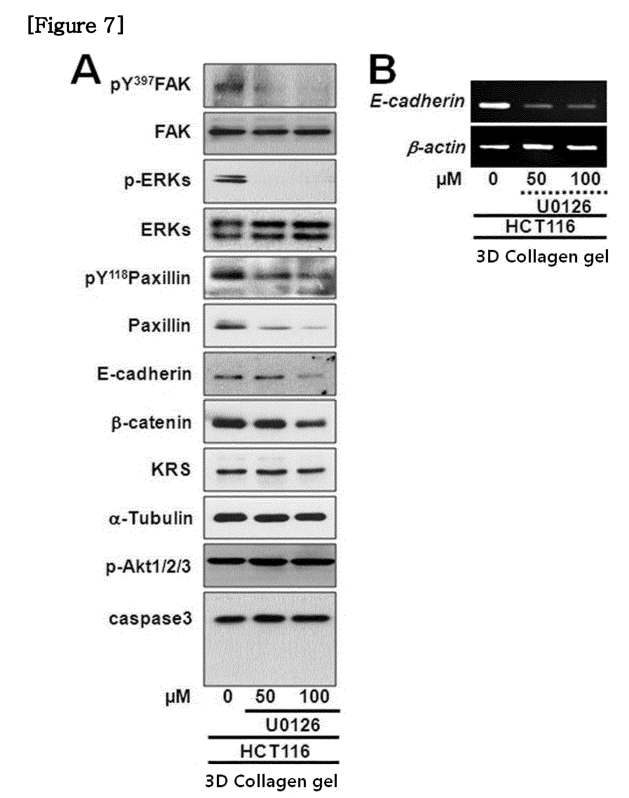

When KRS expressing HCT116 parental cells were treated with ERKs inhibitor, the expressions and activities of FAK, paxillin, and E-cadherin were significantly reduced (see Figure 7A). The mRNA level of signaling activator was also investigated. As a result, it was confirmed that the expression of E-cadherin was significantly reduced by ERKs inhibitor (see Figure 7B).

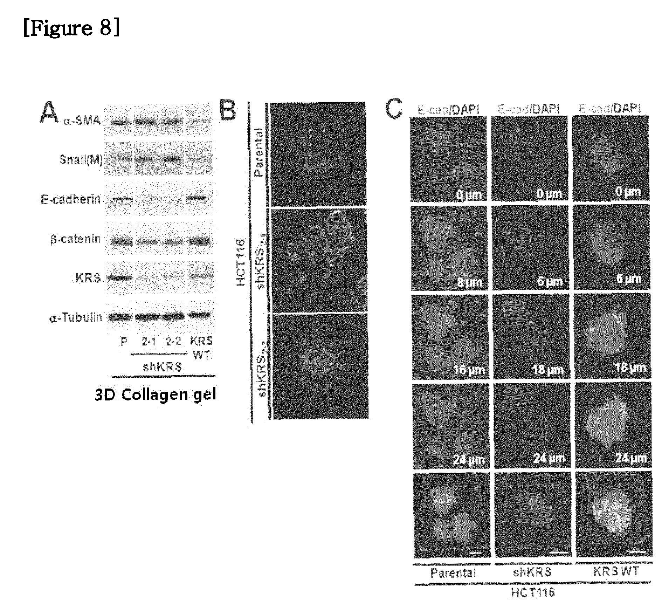

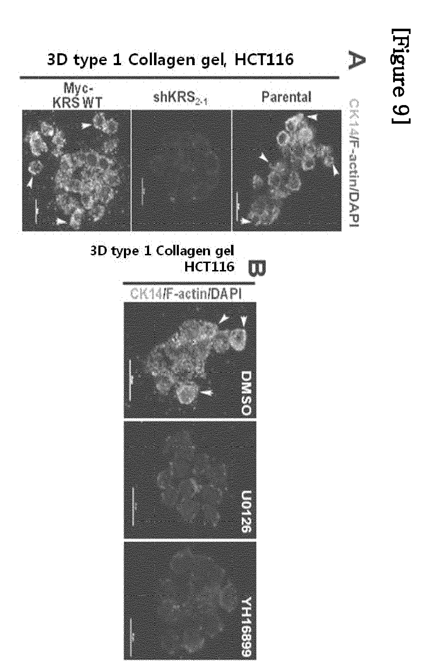

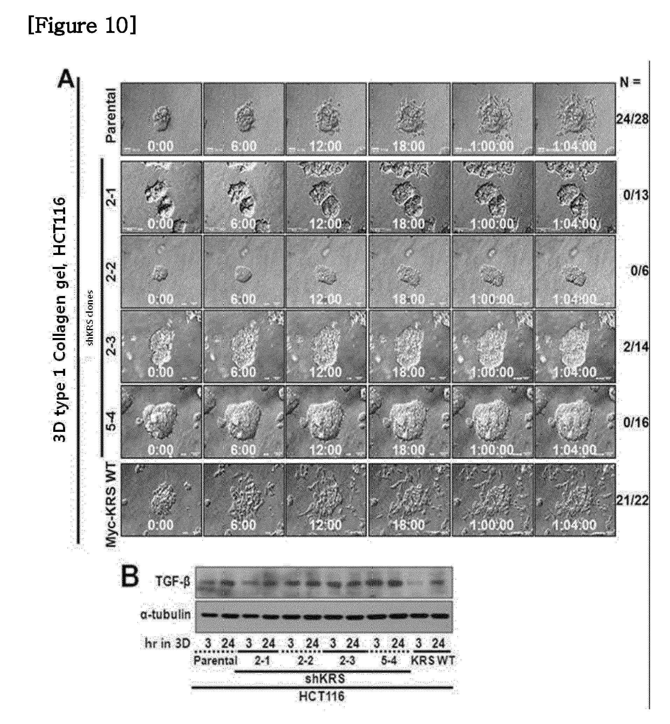

In the spheroidically aggregated cell line expressing KRS cultured in a three-dimensional collagen gel environment, the epithelial marker proteins were down-regulated but the mesenchymal marker proteins were up-regulated (see Figures 8A and B), and also the cell adhesion was not scattering (see Figure 8C). Immunostaining was performed with all of HCT116 parental cells, KRS knock-out cell line (shKRS₂₋₁), KRS over-expressing cell line (myc-KRS WT), and the cell line treated with U0126 and YH16899 cultured in a three-dimensional collagen gel environment for a day in order to measure the expression of the epithelial marker cytokeratin 14 (CK14). As a result, it was confirmed that the expression of CK14 was high in those cells disseminated from the KRS expressing spheroid (arrow), but the expression of CK14 was significantly suppressed in the KRS knock-out cell line or the cell line treated with KRS inhibitor, compared with the parental cells or myc-KRS WT cell line (Figure 9). When cells were cultured in the same culture condition, the dissemination of cells from aggregated cells was observed time dependently in the cells that could express KRS, suggesting that cell migration and invasion were confirmed therein (see Figure 10A).

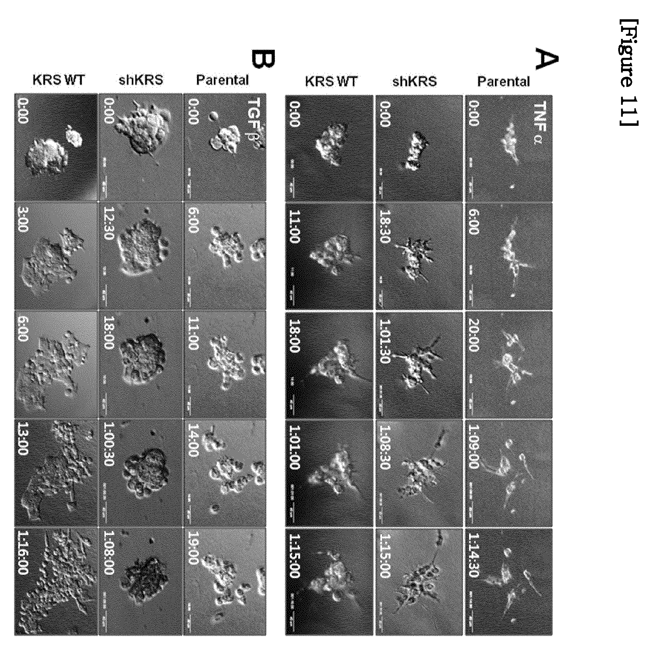

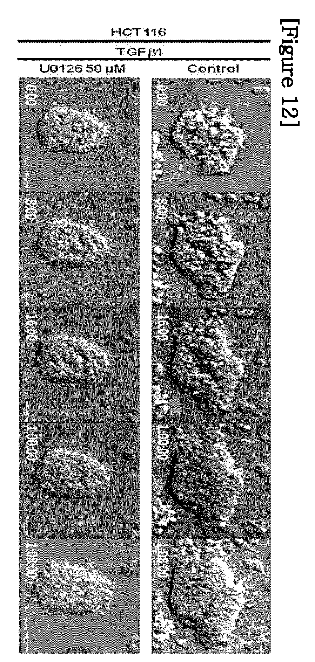

In the spheroidically aggregated cell line expressing KRS cultured in a three-dimensional collagen gel environment, TFGβ1 expression was increased time dependently (see Figure 10B). When the cell line cultured under the condition above was treated with TNFα, cell migration and invasion were observed regardless of KRS expression (see Figure 11A). However, when the cell line was treated with TGFβ1, the dissemination of cells from aggregated cells of the KRS expressing cell line was observed but not in the KRS knock-out cell line (see Figure 11B). In the spheroidically aggregated cell line expressing KRS cultured in a three-dimensional collagen gel environment, the treatment of ERKs inhibitor did not cause cell migration and dissemination (see Figure 12).

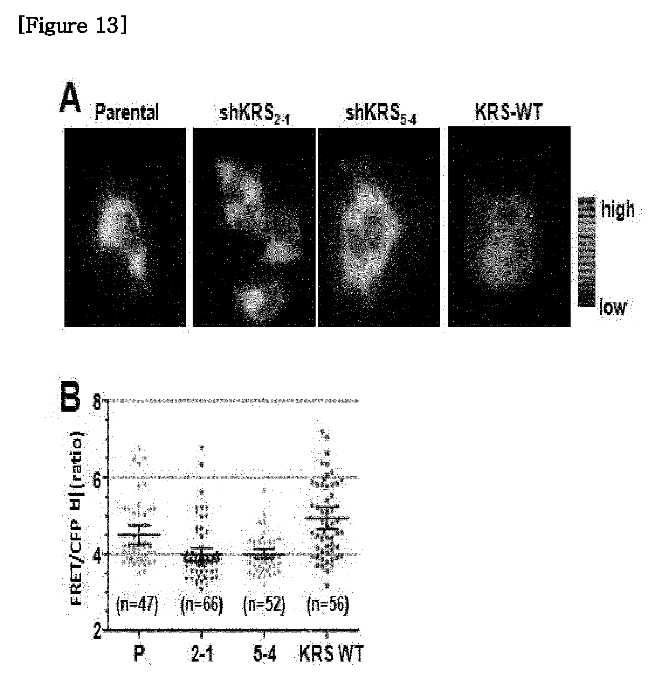

HCT116 cells were transfected with ERK biosensor, followed by investigation of intracellular ERKs activity by FRET. As a result, ERKs phosphorylation was inhibited in the KRS knock-out cell line but was increased in the KRS over-expressing cell line (Figures 13A and 13B).

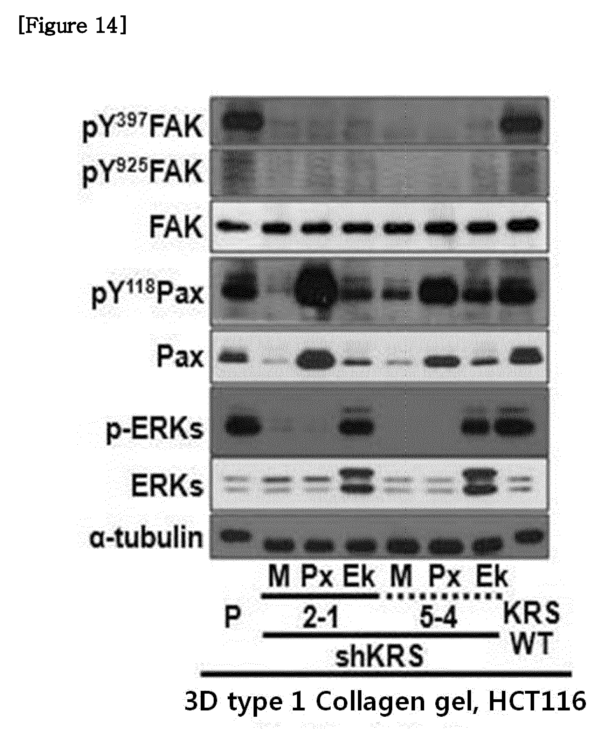

The cell lines wherein KRS expression was suppressed were transiently transfected for 48 hours respectively with pCMV-Mock (M), pCMV-paxillin(paxillin) (Px), and pCMV-ERK1/2 (Ek), followed by Western blotting. As a result, in the cell lines wherein KRS expression was suppressed, the expression of ERK1/2 caused the increase of paxillin expression and Tyr118 phosphorylation. That is, the inhibition of paxillin expression and phosphorylation in the KRS knock-out cells was attributed to the decrease of ERKs activity (Figure 14).

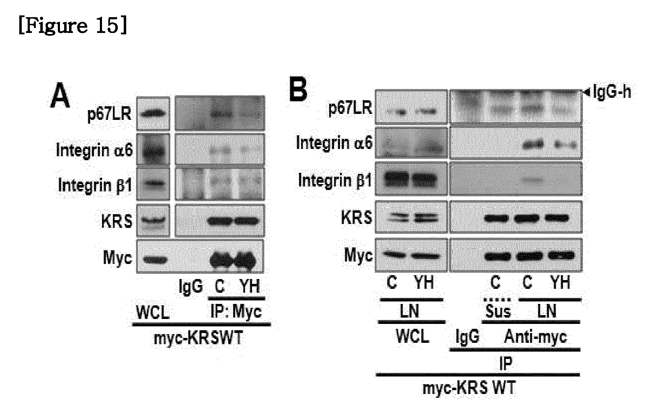

Co-immunoprecipitation was performed with myc-KRS over-expressing HCT116 cell line in a normal two-dimensional condition with 10% serum (Figure 15A) or in laminin (10 mg/ml) coated plate with 2% serum (Figure 15B). As a result, it was confirmed that integrin α6, integrin β1, and p67LR were linked together. In the meantime, integrin α6 and integrin β1 were only conjugated in the normal cell adhesion condition. When the cells were treated with YH16899 (YH), the link was very weak. The conjugation between KRS and p67LR was observed in adhesion signal free suspension but was suppressed by the treatment of YH16899.

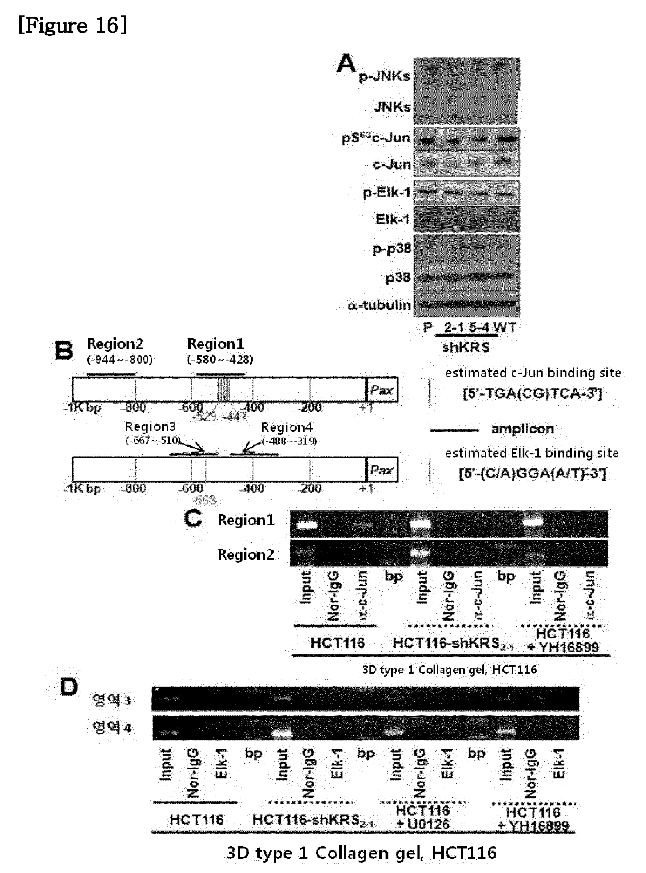

ChIP (Chromatin Immunoprecipitation) was performed with the spheroid cultured in a three-dimensional collagen gel environment. As a result, it was confirmed that KRS, rather than JNKs or p38, dependently activated ERKs regulated the transcription of paxillin through the downstream transcription factor c-Jun (Figures 16A, 16B, 16C, and 16D).

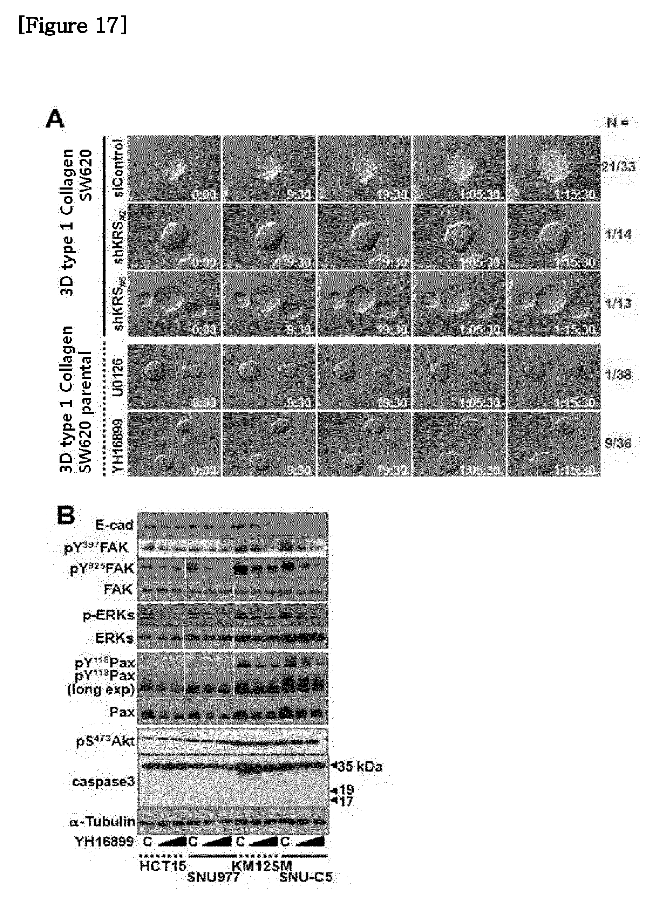

When KRS expression was inhibited in various colorectal cancer cell lines (Figure 17A) or the KRS inhibitor YH16899 was treated to the cell lines (Figure 17B), the typical phenomenon in HCT116 cells such as the dissemination of cells from the spheroid in a three-dimensional collagen gel (Figure 17A) and ERK phosphorylation and the expression and phosphorylation of paxillin were all inhibited (Figure 17B).

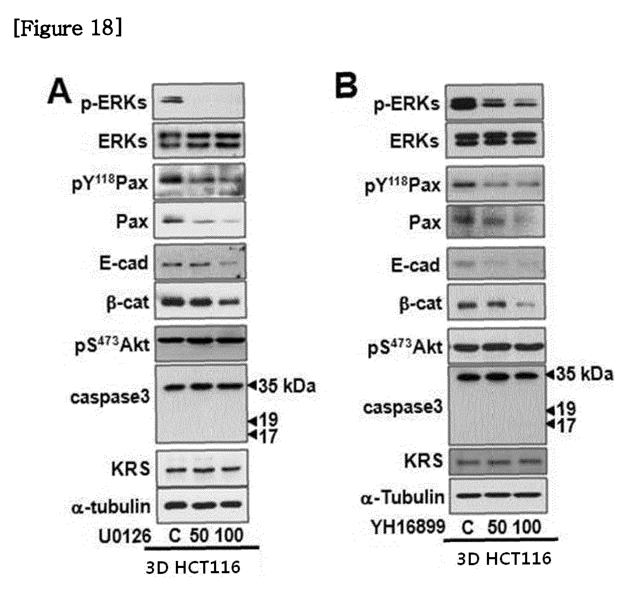

Also, HCT116 cell line was treated with the ERK inhibitor U1026 and the KRS inhibitor YH16899. As a result, the expressions of E-cadherin and β-catenin, the focal adhesion related molecule and the epithelial marker, were decreased and at the same time ERKs phosphorylation and paxillin expression and phosphorylation were also inhibited (Figures 18A and 18B).

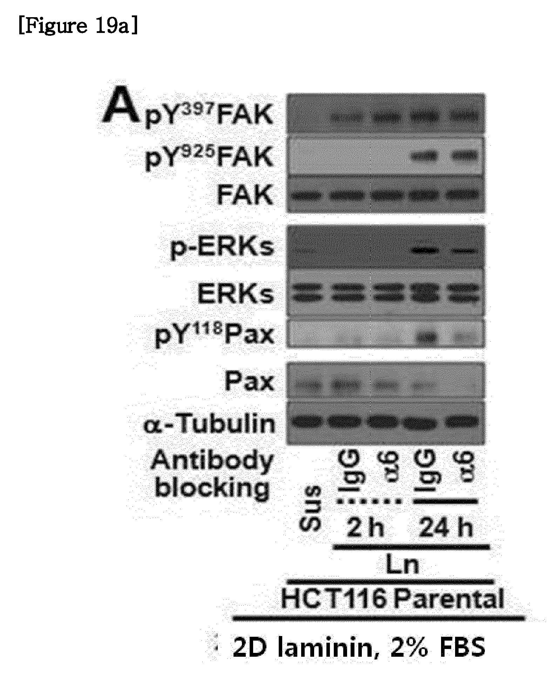

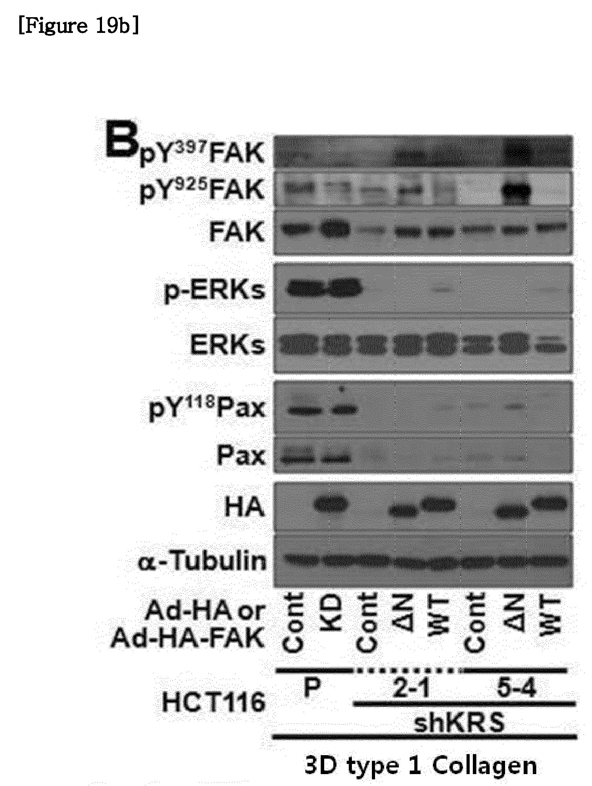

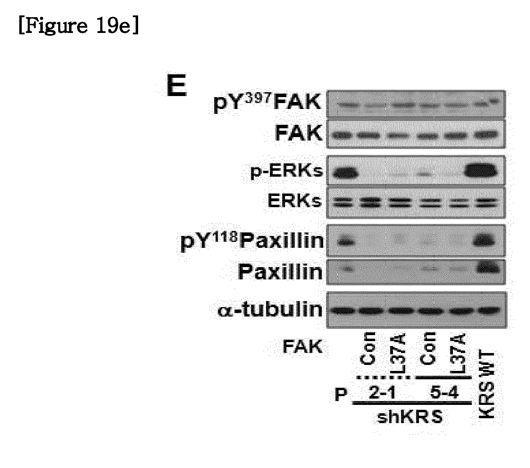

It was also confirmed that the dissemination from the spheroid of KRS expressing parental cells cultured in a three-dimensional collagen gel was not attributed to FAK activation or FAK Tyr 925 phosphorylation mediated ERKs activation, either (Figures 19A, 19B, 19C, 19D, and 19E).

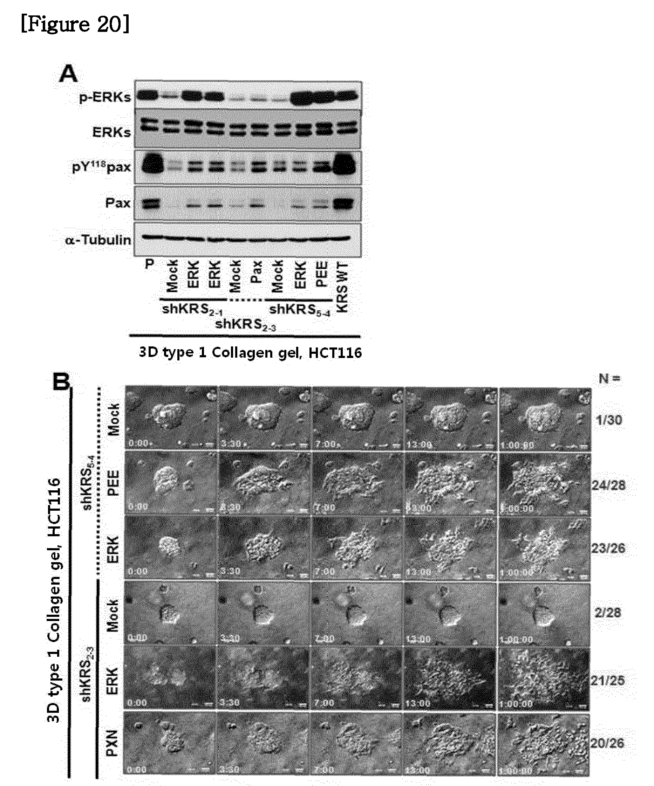

HCT116 KRS knock-out cell line was transfected respectively with pCMV-Mock, pCMV-paxillin, and pCMV-ERK1/2 in order to construct stable cell lines, followed by Western blotting to investigate the expressions and phosphorylations of those molecules (Figure 20A). Spheroids of the cell lines were obtained, which were distributed in a three-dimensional collagen gel, followed by observation under time-lapse microscope. As a result, the activities of paxillin and ERK1/2 were recovered in the KRS knock-out HCT116 cell line in a three-dimensional collagen gel, suggesting that the major signaling activity involved in dissemination was recovered, resulting in the observation of the dissemination (Figure 20B).

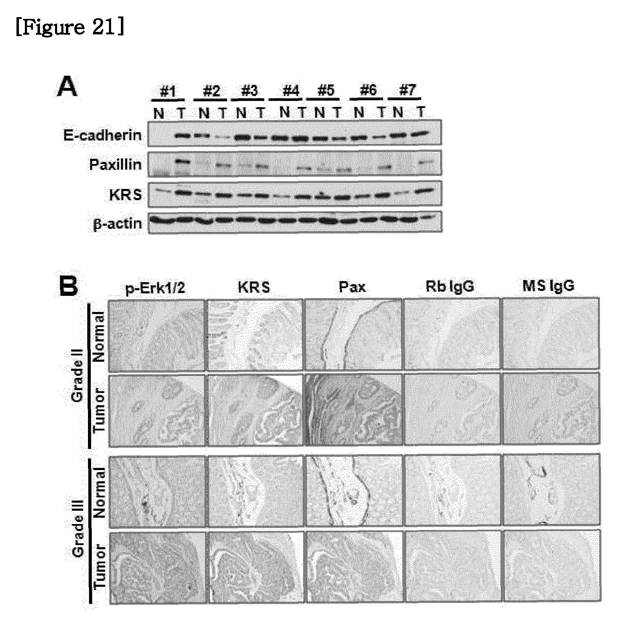

Western blotting (Figure 21A) or immunohistochemical staining (Figure 21B) was performed with paxillin, p-ERKs, and KRS of the extract or tissues obtained from human colon tumor tissues including clinical tissues diagnosed with Grade II and III cancer. As a result, their correlation was confirmed in invasive cancer margin.

Therefore, the present inventors verified that the analysis of the expressions and activities of the active factors involved in cell-cell adhesion, cell-extracellular matrix (ECM) adhesion, cell adhesion signaling activity, and cell dissemination in the colorectal cancer cell line cultured in a three-dimensional or extracellular matrix surrounding environment can be efficiently used for the method for screening a metastasis inhibitor and the method for monitoring cell migration, invasion, and metastasis.

Practical and presently preferred embodiments of the present invention are illustrative as shown in the following Examples.

However, it will be appreciated that those skilled in the art, on consideration of this disclosure, may make modifications and improvements within the spirit and scope of the present invention.

### Example 1: Increase of E-cadherin expression in spheroid cells

### <1-1> Culture of spheroid cells

HT116 (American Type Culture Collection, USA), the human colorectal cancer cell line, was cultured in a 37°C incubator for two days, and then the cells were collected by treating trypsin/EDTA. The collected cells were precipitated by centrifugation, to which 10 mℓ of RPMI-1640 supplemented with FBS was added. Cell number was measured by using hematocytometer. 10 mℓ of RPMI-1640 supplemented with FBS was distributed in a culture dish, to which the above cells were added at the density of 3 x 10⁵. The cells were cultured by hanging drop culture method in a 37°C incubator by using Perfecta3D Hanging Drop Plates (3D Biomatrix, USA) to obtain spheroidically aggregated cells.

As a result, as shown in Figure 1B, the morphology of those cells cultured in a two-dimensional environment was flat shape adhered onto the floor and when the cells were cultured by hanging drop culture, they formed spheroidically aggregated cells (Figure 1B).

### <1-2> Increase of E-cadherin expression in the spheroidically aggregated cells

To investigate the expression of E-cadherin in the spheroidically aggregated cells obtained by the method of Example <1-1>, Western blotting was performed.

Particularly, the spheroidically aggregated cells proceeded to spin-down at 100 rpm to gather them together, to which 100 *µ*ℓ, of lysis buffer [50 mM Tris-HCl, pH 7.4, 1% NP-40, 0.25% sodium deoxycholate, 150 mM NaCl, 1 mM EDTA] supplemented with 1% SDS, Na₃O₄V, and protease inhibitor cocktails (GenDepot, USA) was added, followed by reaction at 4°C for at least 1 hour. The cells were collected, followed by centrifugation at 4°C at 13000 rpm for 30 minutes. The supernatant was transferred in a new microcentrifuge tube, followed by protein quantification using BCA reagent (Thermo Scientifics, USA). To the quantified sample was added 4x sample buffer [100% glycerol 4 mℓ, Tris-HCl, pH 6.8, 2.4 mℓ, SDS 0.8 g, bromophenol blue 4 mg, β-mercaptoethanol 0.4 mℓ, H₂O 3.1 mℓ (total 10 mℓ)], which was boiled at 100°C for 5 minutes. The sample proceeded to 12% SDS-PAGE electrophoresis. The electrophoresis product was transferred on Protran™ nitrocellulose membrane (Whatman), which was then pre-treated with 5% skim milk for 1 hour. After the pre-treatment, the membrane was washed with PBS (130 mM NaCl, 13 mM Na2HPO4, 3.5 mM NaH2PO4, pH 7.4) twice, followed by reaction with the primary antibodies of E-cadherin (Santa Cruz Biotech, USA) and α-tubulin (Sigma, USA) at 4°C for 15 hours. On the next day, reaction with the secondary antibody was induced, followed by development on x-ray film using ECL (Pierce, USA).

As a result, as shown in Figure 1C, the expression of E-cadherin was increased over the culture time in the spheroidically aggregated cells (Figure 1C).

### Example 2: Epithelial-to-mesenchymal transition in the spheroidically aggregated cell line wherein lysyl-tRNA synthetase (KRS) expression was suppressed

### <2-1> Construction of the spheroidically aggregated cell line wherein KRS expression was over-expressed or suppressed

The spheroidically aggregated cell line wherein KRS expression was over-expressed or suppressed was constructed from the spheroidically aggregated cells obtained by the method of Example <1-1>.

Particularly, the cell line wherein KRS expression was suppressed was constructed by transfecting cells with lysyl-tRNA synthetase MISSION® shRNA plasmid DNA (Sigma, USA). At this time, the said KRS was homo sapiens lysyl-tRNA synthetase (transcript variant 1, mRNA sequence: NM_001130089.1). This KRS includes 1219 bp long exon 1 ∼ 15. Among the purchased KRS shRNA plasmid DNAs, shKRS-0 (SEQ. ID. NO: 1) targets 1581 ∼ 1604 bp (exon 12) of KRS, shRKS-1 (SEQ. ID. NO: 2) targets 437 ∼ 459 bp (exon 3 ∼ 4), shKRS-2 (SEQ. ID. NO: 3) targets 911 ∼ 933 bp (exon 7), and shKRS-5 (SEQ. ID. NO: 4) targets 1071 ∼ 1092 bp (exon 8). The sequences of shRNA used herein are shown in Table 1. TRC1.5-pLK0.1-puro was used as the expression vector. Selection was performed by using puromycin to establish stable cell lines. ShKRS-0, shKRS-2, and shKRS-5 which all show the KRS inhibiting effect are targeting class II core domain that forms a dimer to play a role as a bridge to connect lysine to a corresponding tRNA ribose 3'OH to help smooth protein synthesis.

**[Table 1]**

| shKRS | Sequence (5'→3') | SEQ. ID. NO |
|---|---|---|
| shKRS-0 | CCGGCCTGGAAGTGACTTGCATCAACTCGAGTTGATGCA AGTCACTTCCAGGTTTTTG | 1 |
| shKRS-1 | CCGGCGTGGACCCAAATCAATACTACTCGAGTAGTATTG ATTTGGGTCCACGTTTTTG | 2 |
| shKRS-2 | CCGGCCAGAGATACTTGGACTTGATCTCGAGATCAAGTC CAAGTATCTCTGGTTTTTG | 3 |
| shKRS-5 | CCGGGCCTTTCATCACTTATCACAACTCGAGTTGTGATA AGTGATGAAAGGCTTTTTG | 4 |

For the construction of the KRS over-expressing cell line, myc labeled KRS sequence was cloned (Kim et al., FASEB J. 2012 Oct; 26 (10) : 4142-59). The cloned pcDNA-Myc-KRS expression vector was introduced in cells, which were treated with 250 *µ*g/mℓ, of G418, resulting in the construction of the myc labeled KRS over-expressing cell line.

As a result, the KRS knock-out cell lines were constructed by using shKRS-2 and shKRS-5 shRNA, wherein the three clones established by using shKRS-2 were named 2-1, 2-2, and 2-3, and the clones obtained by using shKRS-5 was named 5-4.

### <2-2> Inhibition of KRS expression in the established spheroidically aggregated cell line

Western blotting was performed to investigate whether or not the KRS expression was inhibited in the spheroidically aggregated cells of the KRS knock-out cell lines constructed by the method of Example <2-1>. Particularly, Western blotting was performed by the same manner as described in Example <1-2> by using the primary antibodies of KRS (Abcam, Great Britain) and α-tubulin (Sigma, USA).

As a result, as shown in Figure 1E, it was confirmed that the KRS expression was inhibited in the spheroidically aggregated cell line treated with shKRS.

### <2-3> The epithelial-to-mesenchymal transition (EMT) related marker gene mRNA expression in the spheroidically aggregated KRS knock-out cell line

Reverse transcription PCR was performed to investigate the expression level of EMT related marker gene mRNA in the KRS knock-out cell line confirmed by the method of Example <2-2>.

Particularly, mRNA was extracted from the cell line established by the method of Example <2-1> by using TRizol (Invitrogen, USA), followed by quantification using nano drop (Thermo Scientific, USA). The quantified mRNA was synthesized as cDNA by using Amfirivert cDNA Synthesis Master Mix (GenDePot, USA). PCR was performed by using ThermoScientific DreamTaq Green PCR Master Mix (Thermo Scientific, USA) and β-actin as a primer. The quantity of each sample was measured by using β-actin. EMT marker mRNA was quantified by using the primers listed in Table 2.

**[Table 2]**

| Primer | Sequence (5'→3') | SEQ. ID. NO |
|---|---|---|
| hEcad-5 | TGCCCAGAAAATGAAAAAGG | 5 |
| hEcad-3 | GTGTATGTGGCAATGCGTTC | 6 |
| hNcad-5 | ACAGTGGCCACCTACAAAGG | 7 |
| hNcad-3 | CCGAGATGGGGTTGATAATG | 8 |
| hVim-5 | GAGAACTTTGCCGTTGAAGC | 9 |
| hVim-3 | GCTTCCTGTAGGTGGCAATC | 10 |
| hTwist-5 | GGAGTCCGCAGTCTTACGAG | 11 |
| hTwist-3 | TCTGGAGGACCTGGTAGAGG | 12 |

As a result, as shown in Figure 1F, the epithelial marker E-cadherin mRNA was significantly down-regulated in the KRS knock-out cell line, compared with the expression levels in the normal HCT116 cell line and KRS over-expressing cell line. On the contrary, the mesenchymal markers Vimentin, N-cadherin, and twist mRNAs were significantly up-regulated (Figure 1E).

### <2-4> The epithelial-to-mesenchymal transition (EMT) related marker gene protein expression in the spheroidically aggregated KRS knock-out cell line

Western blotting was performed to investigate the expression level of EMT related marker gene protein in the KRS knock-out cell line confirmed by the method of Example <2-2>. At this time, the primary antibodies of E-cadherin (Santa Cruz Biotech, USA), vimentin (Sigma, USA), KRS (Abcam, Great Britain), and α-tubulin (Sigma, USA) were used.

As a result, as shown in Figure 1G, it was confirmed that the expression of E-cadherin in the KRS knock-out cell line was significantly reduced but the expression of vimentin therein was increased (Figure 1G). So, it was suggested that the inhibition of KRS expression could cause partial EMT and accordingly reduce cell-cell adhesion, by which cell migration and invasion would be also affected.

### Example 3: Inducement of incomplete EMT phenotype by the inhibition of KRS expression in a two-dimensional culture environment

### <3-1> Decrease of the cell-cell adhesion or EMT related protein expression by the inhibition of KRS expression in a two-dimensional culture environment

Western blotting was performed to investigate the cell-cell adhesion or EMT related protein expression in HCT116 cells cultured in 100 mm cell culture dish in a two-dimensional environment with 10% serum.

Particularly, the cell line cultured in a two-dimensional environment with 10% serum was washed with PBS twice, followed by reaction at 4°C for 15 minutes in 200 *µ*ℓ of lysis buffer [50 mM Tris-HCl, pH 7.4, 1% NP-40, 0.25% sodium deoxycholate, 150 mM NaCl, 1 mM EDTA] supplemented with SDS, Na₃O₄V, and protease inhibitor cocktails (GenDepot, USA). The cells were collected and centrifuged at 4°C at 13000xg for 30 minutes. The supernatant was transferred in a new microcentrifuge tube, followed by protein quantification using BCA reagent (Thermo Scientifics, USA). To the quantified sample was added 4x sample buffer [100% glycerol 4 mℓ, Tris-HCl, pH 6.8, 2.4 mℓ, SDS 0.8 g, bromophenol blue 4 mg, β-mercaptoethanol 0.4 mℓ, H₂O 3.1 mℓ (total 10 mℓ)], which was boiled at 100 °C for 5 minutes. The prepared sample was treated with the primary antibodies of ZO-1 (Zymed, USA), β-catenin (Santa Cruz, USA), and E-cadherin by the same manner as described in Example <1-2>.

As a result, it was confirmed that the expressions of the epithelial marker proteins ZO-1 (Zymed), β-catenin (Santa Cruz), and E-cadherin (Santa Cruz) that can be an index to measure cell-cell adhesion were all reduced in the KRS knock-out cell lines, compared with the normal HCT116 cells and the KRS over-expressing cell line. On the contrary, the expressions of the mesenchymal marker proteins, Vimentin (Sigma), N-cadherin (BD Sciences), twist1 (Abcam), snail1 (Cell signaling), and fibronectin (DAKO) were all increased in the KRS knock-out cell lines. The expression levels of Integrin α6 (Chemicon), Integrin β1 (Santa Cruz), Integrin β4 (Santa Cruz), p67 laminin receptor (Abcam), and laminin (Abcam) were not changed and as consistent as usual in spite of the regulation of KRS expression (Figure 2A).

### <3-2> Effect of KRS inhibition on the surrounding microenvironmental factors in a two-dimensional culture environment

To observe the surrounding microenvironmental factors in a two-dimensional culture environment over the time, the culture fluid supplemented with 10% FBS was additionally treated with such cytokines as TNFα and TGFβ, followed by investigation of the expression of the epithelial marker protein that can be an index for cell-cell adhesion by the same manner as described in Example <3-1>.

As a result, as shown in Figure 2B, the expression of the protein was not changed by the treatment of TNFα in the normal HCT116 and the KRS over-expressing cell line. However, when TGFβ1 was treated thereto, occludin or ZO-1, involved in cell-cell adhesion, was down-regulated but the mesenchymal marker protein, fibronectin, was up-regulated. In the meantime, when the KRS knock-out cell line was treated with TGFβ1, the epithelial marker protein like occludin was up-regulated but fibronectin was down-regulated (Figure 2B).

Therefore, it was confirmed that the KRS expression was involved in EMT in cells. Precisely, KRS seems to be positively involved in cell migration and invasion with the support of the microenvironmental factors such as TGFβ1.

### <3-3> E-cadherin inhibition and cell-cell adhesion by KRS inhibition in a two-dimensional culture environment

The inhibition of E-cadherin expression by the suppression of KRS expression was confirmed by immunofluorescence by the same manner as described in Example <3-1>.

Particularly, the HCT116 cells cultured in a 37°C CO₂ incubator for 2 days were detached by treating trypsin/EDTA. The cell number was counted by using hematocytometer. Then, the cells were distributed in a 6-well plate equipped with cover glass at the density of 1 x 10⁶ cells/well. The cells were cultured in a 37°C CO₂ incubator for one day. The cells were fixed in 4% formaldehyde for 30 minutes and then treated with 30 mM glycine for 10 minutes. The cells proceeded to permeabilization for 5 minutes with 0.5% triton X-100, followed by pre-treatment with 2% BSA for 1 hour. The cells were treated with FITC-labeled E-cadherin primary antibody (Bio Legend, USA) for 16 ∼ 18 hours at 4°C. Lastly, the cells were treated with DAPI (4',6-diamidino-2-phenylindole, blue) solution for staining nuclei, leading to DAPI staining. Upon completion of the staining, the cover glass where the stained cells were attached was placed on the slide glass, followed by mounting. The stained cells were observed under fluorescent microscope (Olympus, Japan).

As a result, as shown in Figure 2C, the normal expression of E-cadherin was observed in the cell adhesion region in the normal HCT116 cells and the KRS over-expressing cell line, while E-cadherin expression was inhibited in the cell adhesion region when KRS expression was suppressed. However, cell-cell binding or adhesion was not affected. The test with β**-**catenin that is another marker usable for the confirmation of cell-cell adhesion displayed the consistent result (Figure 2C). As shown in Figure 2D, the KRS knock-out cell lines (shKRS₂₋₁, shKRS₅₋₄) were compared with the KRS expressing HCT116 parental cells and the KRS over-expressing cell line (Myc-KRS-WT). As a result, unlike the pattern of the EMT (epithelial-mesenchymal transition) marker protein expression, cell/cell contact was not changed in shKRS₂₋₁ and shKRS₅₋₄, and accordingly the cells formed a colony normally (Figure 2D). Despite the EMT marker protein expression has been changed in a two-dimensional environment, the morphology and shape of those cells growing on the plate were not changed, suggesting that the KRS expression did not cause complete EMT but cause incomplete EMT.

### Example 4: Investigation of cell adhesion signaling factors in the cell line wherein KRS has been regulated to be expressed or unexpressed in a laminin (10 µg/ml) pre-coated two-dimensional culture environment

It was previously reported that KRS was involved in the signaling activity relating to cell migration laminin-dose dependently after KRS has moved into plasma membrane from cytoplasm (Kim DG et al., FASEB J. 2012 Oct;26(10):4142-59). So, in order to investigate the correlation between KRS expression and cell-ECM adhesion, the inventors prepared cell suspension for 1 hour, which was then re-distributed onto the laminin coated culture dish. The KRS mediated cell adhesion related signaling activity was investigated by measuring the expressions and phosphorylations of those proteins relating to focal adhesion such as FAK, Src, paxillin, and ERKs.

Particularly, HCT116 parental cells, KRS knock-out cell line, and KRS over-expressing cell line were separated from culture dish, followed by centrifugation to separate the cells from the culture solution. Since then, the replacing buffer prepared with serum-free RPMI1640 medium supplemented with 2% FBS (to obtain healthy cells with minimizing the effect of serum) and 1% BSA had been used as the culture medium. The cell number was counted by using hemocytometer then the replating buffer was additionally added thereto to make the cell density to be 0.2 × 10⁶ cells or 2 × 10⁶ cells, followed by distribution in e-tube. Total volume was adjusted to be 1 ml with filling it with the replating buffer. HCT116 parental cells that had been treated with YH16899 were mixed with the replating buffer (final conc.: 10 µM). The suspension was rolled at 37°C for 1 hour. The culture dish that had been pre-coated with laminin (10 µg/ml) for the past overnight and the cover glass were washed with PBS twice, to which the replating buffer was added, which stood at 37°C. Cells were mixed for 1 hour for immunofluorescence. Precisely, the cells were distributed in the 6-well plate containing the laminin coated cover glass at the density of 0.2 X 10⁶ cells/well, and distributed in the laminin coated 60 mm dish prepared for Western blotting at the density of 2 X 10⁶ cells, followed by culture in a 37°C incubator for 2 hours. The sample for immunofluorescence was washed with PBS twice, followed by fixing in 4% formaldehyde for 15 minutes. Then, the cells were treated with 30 mM glycine for 10 minutes, followed by permeabilization with 0.5% triton X-100 for 5 minutes. Then, blocking was performed with 3% BSA for 1 hour. The unlabeled p-ERKs (Cell signaling), p-Tyr118-paxillin (Santa Cruz Biotech. Inc.), or p-Tyr397-FAK (Abcam) antibody was treated thereto at 4°C for 16 ∼ 18 hours, and the secondary antibody was treated thereto at room temperature for 2 hours. After performing actin staining using phalloidin, DAPI (4',6-diamidino-2-phenylindole; blue) was treated thereto for nuclei staining. The sample for Western blotting was washed with PBS twice, and then protein was extracted by treating lysis buffer (50 mM Tris-HCl, pH7.4, 1% NP-40, 0.25% sodium deoxycholate, 150 mM NaCl, 1 mM EDTA) thereto. The protein was quantified and then the prepared sample proceeded to Western blotting.

As a result, the phosphorylations of Src, ERKs, and paxillin according to cell adhesion were significantly reduced in the KRS knock-out cell lines (shKRS₂₋₁, shKRS₅₋₄), compared with the KRS expressing parental cell line and the KRS over-expressing cell line (Myc-KRS-WT). However, the phosphorylation of FAK was not changed (Figure 3A). The phosphorylations of ERKs and paxillin, suggested to be dependent on KRS, were therefore confirmed to be induced by the KRS signaling activity without being mediated by FAK. Immunostaining was performed with the focal adhesion related proteins such as p-ERKs and paxillin. As a result, the phosphorylation levels of ERKs and paxillin were high in the KRS expressing parental cells and the KRS over-expressing cell line, but were reduced in the KRS knock-out cell lines, which was consistent with the result of Western blotting (Figures 3B and 3C). When the cells were treated with 10 µM of YH16899 that is the inhibitor suppressing the conjugation between KRS and laminin receptor (Kim DG et al., Nat Chem Biol. 2014 Jan;10(1):29-34.), the phosphorylation levels of Scr, ERKs, and paxillin were reduced in the KRS expressing parental cells and the KRS over-expressing cell line treated with the inhibitor, compared with the control not treated with the inhibitor (Figure 3A). The result of immunofluorescence with p-EKRs and paxillin was also consistent (Figures 3E and 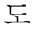 3F). The above results verify that KRS plays an important role in the cell-ECM adhesion related signaling factor activity. However, the treatment of KRS inhibitor or YH16899 to HCT116 did not cause any change in the phosphorylation of Tyr397 (Figure 3B), and likewise cell morphology or spreading was not much different, either (Figures 3D and 3G).

### Example 5: Investigation of the suppression of cell-extracellular matrix (ECM) adhesion related signaling activity by the inhibition of KRS suppression in a two-dimensional culture environment

Western blotting was performed to investigate cell-ECM adhesion and the adhesion related signaling activity in the cell line established by the same manner as described in Example <2-1> in a two-dimensional culture environment. Cells were re-distributed in the specific ECM pre-coated culture vessel (serum free) for investigation of the cell-ECM adhesion signaling activity. Within two hours, the cell adhesion related signaling factor activity was confirmed. At this time, the investigation of the signaling activity was performed by the conventional method well known to those in the art (Juliano et al., 2001).

Particularly, At this time, the primary antibodies of pY³⁹⁷FAK (Abcam, Great Britain), pY⁵⁷⁷FAK (Santa Cruz, USA), pY⁸⁶¹FAK (Santa Cruz, USA), pY⁹²⁵FAK (Santa Cruz, USA), FAK (Santa Cruz, USA), p-ERKs (cell signaling, USA), ERKs (cell signaling, USA), KRS (Abcam, Great Britain), pY⁴¹⁶c-Src (cell signaling, USA), c-Src (Santa Cruz, USA), pY¹¹⁸paxillin (Santa Cruz, USA), paxillin (BD Transduction Laboratories, USA), and α-tubulin (Sigma, USA) were used.

KRS expressing HCT116 parental cell line, KRS knock-out cell lines (shKRS2-1 and shKRS5-4), and KRS over-expressing cell line in the serum free culture fluid supplemented with 1% BSA were re-distributed in the fibronectin (10 *µ*g/mℓ) pre-coated culture dish. Two hours later, cell extracts were obtained, followed by Western blotting by the same manner as described in Example <1-2>.

As a result, as shown in Figure 4A, the phosphorylations of FAK and ERKs were observed in the KRS expressing HCT116 parental cell line and the KRS over-expressing cell line but the phosphorylations of FAK and ERKs were significantly reduced in the KRS knock-out cell line (Figure 4A). As shown in Figure 4B, the phosphorylations of c-Src and paxillin were significantly reduced in the KRS knock-out cell line, compared with the HCT116 parental cell line, and particularly the suppression of paxillin expression was more significant (Figure 4B). As shown in Figure 4C, the phosphorylation of FAK was just a little reduced in the KRS knock-out cell line, compared with the KRS expressing parental cell line and the KRS over-expressing cell line, but the phosphorylation of ERK was significantly reduced (Figure 4C). The above results indicate that KRS plays an important role in the cell-ECM adhesion related signaling factor activity.

### Example 6: Inhibition of focal adhesion by the suppression of KRS expression in a two-dimensional culture environment

Immunofluorescence was performed to investigate whether or not the cell-ECM adhesion mediated focal adhesion formation was dependent on KRS expression.

Particularly, KRS expressing HCT116 parental cell line, KRS knock-out cell lines (shKRS₂₋₁ and shKRS₅₋₄), and KRS over-expressing cell line in the serum free culture fluid supplemented with 1% BSA were re-distributed in the laminin (10 *µ*g/mℓ) or fibronectin (10 *µ*g/mℓ) pre-coated cover glass. Two hours later, immunofluorescence was performed by the same manner as described in Example <3-3>. At this time, the Tyr³⁹⁷ phosphorylated FAK primary antibody (pY³⁹⁷FAK) and DAPI were used to stain nuclei.

As a result, as shown in Figure 3D, pY³⁹⁷FAK rich focal adhesion was fully developed in the HCT116 parental cell line and the KRS over-expressing cell line in the fibronectin coated two-dimensional culture environment, while pY³⁹⁷FAK was not so well-stained as spots in the KRS knock-out cell line that focal adhesion was believed to be significantly suppressed (Figure 4D). The above results indicate that KRS plays an important role in cell-ECM adhesion. However, in the two-dimensional environment that had been pre-treated with laminin which is necessary for KRS to be functioning, pERKs and p-Tyr118 paxillin spots were reduced in the cells wherein KRS expression was suppressed or KRS inhibitor was treated. In the meantime, the location of the Tyr³⁹⁷ phosphorylated FAK and the related cell morphology were not different by the KRS expression or by the treatment of KRS inhibitor. Therefore, it was confirmed that the KRS expression plays an important role in cell-extracellular matrix (ECM) adhesion signaling including ERKs and paxillin.

### Example 7: Inhibition of the expressions and activities of ERKs and paxillin by the suppression of KRS expression in the spheroidically aggregated cells cultured in a three-dimensional collagen gel environment

### <7-1> Culture of HCT116 cells in a three-dimensional collagen gel environment

To culture the spheroidically aggregated cells in a three-dimensional collagen gel environment, 10 x regeneration buffer [2.2 g sodium bicarbonate; 4.8 g HEPES (in 100 mℓ)], 10 x RPMI medium, collagen type I (BD Bioscience, USA), 2 N NaOH, and serum free RPMI-1640 were well mixed, resulting in the solution containing type I collagen (final conc: 2.0 ∼ 2.5 mg/mℓ). All the processes were accomplished on ice to prevent collagen coagulation. The prepared solution was loaded in the magnetic 4-well chamber (Live Cell Instrument, Korea) fitting confocal microscope culture system or PDMS (polydimethylsiloxane) with holes in the diameter of 10 mm. At this time, the collagen solution not containing cells was spread thin on the hole floor to form a collagen bottom layer, to which the cells and collagen mixture were added, followed by solidification in a 37°C incubator for 30 minutes. HCT116 cells were cultured for 2 days until the spheroidically aggregated cells were formed, which were filtered by using cell strainer (SPL, Korea) to eliminate those aggregated cells that were bigger than the size of 70 *µ*m. So, those spheroidically aggregated cells that had passed through 70 *µ*m holes were gathered and washed with serum free PRMI1640 twice. Then, the cells were precipitated by centrifugation. After eliminating the culture medium, the collagen solution was added to the remaining cells, which was well mixed. The mixture was poured in each hole of PDMS, the culture vessel, or the 48-well plate by 80 ∼150 *µ*ℓ, followed by solidification in a 37°C incubator for 30 minutes. When the mixture was fully solidified, RPMI-1640 supplemented with FBS was added thereto.

As a result, as shown in Figure 1D, the spheroidically aggregated cell line was growing bigger over the time, which was observed as dark under optical microscope, suggesting that the cell line was proliferated (Figure 1D).

### <7-2>: Inhibition of the expressions and activities of ERKs and paxillin by the suppression of KRS expression in a three-dimensional collagen gel environment

The importance of KRS in relation to cell-extracellular matrix adhesion was confirmed in the HCT116 cell line cultured in a two-dimensional environment. At this time, it was also confirmed that the phosphorylations of FAK, ERKs, paxillin, and c-Src and the expression of paxillin were significantly reduced by the inhibition of KRS expression. The present inventors performed Western blotting to investigate if the same pattern was observed in the spheroidically aggregated cells in a three-dimensional collagen gel environment.

Particularly, the cell lines established by the same manner as described in Example <2-1> were used herein. KRS expressing HCT116 parental cell line, KRS knock-out cell lines (shKRS₂₋₁, shKRS₂₋₂, shKRS₂₋₃ and shKRS₅₋₄), and KRS over-expressing cell line were cultured by the same manner as described in Example <7-1>. At this time, the cell lines were placed in a three-dimensional collagen gel environment. 3 ∼ 4 hours later, samples were obtained therefrom. Precisely, a tipped-off yellow tip was used to pick up the collagen gel and medium containing the cells cultured in a 48-well plate and they were gathered in a 1.7 mℓ microcentrifuge-tube. The cells were centrifuged at 4°C at 5,000 x g for 1 minute. The supernatant was eliminated and the remaining pellet was washed with cold PBS twice, followed by centrifugation at the same speed for 1 minute as well. To the pellet was added 100 *µ*ℓ of lysis buffer containing 1% SDS, Na₃O₄V, and protease inhibitor cocktail (GenDepot, USA), followed by reaction at 4°C for at least one hour. Centrifugation was performed at 4°C at 13, 000 x g for 30 minutes. The supernatant was transferred in a new microcentrifuge-tube. To the supernatant was added 4 x sample buffer, which was boiled at 100 °C for 5 minutes, resulting in the preparation of the sample. The following process was performed by the same manner as described in Example <1-2>. At this time, the primary antibodies of pY³⁹⁷FAK, pY⁵⁷⁷FAK, FAK, pY⁴¹⁶c-Src, p-ERKs, ERKs, paxillin, KRS, and α-tubulin were used.

As a result, as shown in Figure 5, the phosphorylation of FAK was not specifically reduced in the KRS knock-out cell line cultured in a three-dimensional collagen gel environment, compared with the cell line expressing KRS, and instead the phosphorylation was increased over the culture time in a three-dimensional collagen gel environment, suggesting that FAK phosphorylation did not relate to KRS expression in a three-dimensional collagen gel environment. However, the phosphorylation of EKRs and the expression of paxillin were significantly inhibited in the KRS knock-out cell line in a three-dimensional collagen gel environment. In the KRS over-expressing cell line cultured in a two-dimensional environment, the phosphorylation of ERKs was significantly increased even in the condition of suspension, suggesting that KRS expression closely related to the phosphorylation and activation of ERKS and paxillin expression.

### Example 8: Investigation of the negative effect of KRS expression inhibition, ETK activation inhibition, or KRS function inhibition on ERKs activity and paxillin expression and activation in a three-dimensional (3D) collagen gel environment

To investigate whether or not the KRS dependent changes in focal adhesion related protein expression pattern were observed in the cells cultured in a three-dimensional collagen gel environment, immunostaining was performed with normal HCT116 cells, KRS knock-out cell lines (shKRS₂₋₁, shKRS₅₋₄), and the cell lines treated with the MEK/ERK selective inhibitor U0126 and the KRS inhibitor YH16899 cultured in a three-dimensional collagen gel environment for a day to measure ERKs phosphorylation and paxillin expression, followed by observation under confocal microscope.

Particularly, collagen type I was distributed in PDMS, which was treated with nothing for a day (Figure 6A and Figure 6B) or treated with U0126 (50 mM) or YH16899 (50 mM) (Figure 6C and Figure 6D). Collagen gel containing the cultured cells was fixed in 4% formaldehyde for 30 minutes, followed by the treatment with 30 mM glycine for 30 minutes. Permeabilization was induced by using 0.5% triton X-100 for 30 minutes, followed by blocking with 3% BSA solution for 2 hours. The unlabeled p-ERKs (cell signaling) and paxillin (BD bioscience) antibodies were treated thereto at 4°C at least 16 ∼ 18 hours, followed by the treatment with the secondary antibody for at least 4 hours at room temperature. At last, DAPI (4',6-diamidino-2-phenylindole) solution was treated thereto in order to stain nuclei. The stained cells were observed under confocal microscope.

As a result of p-ERKs immunostaining, stained spots were observed in the inside and surrounding area of nuclei in the normal HCT116 spheroid cells (Figures 6A and 6C). It was also confirmed that paxillin was strongly stained so that paxillin spots were observed in the focal adhesion area (Figures 6B and 6D). However, it was confirmed that the ERKs phosphorylation level was very low (Figures 6A and 6C) and the paxillin immunostaining level was significantly reduced (Figures 6B and 6D) in the spheroids of KRS knock-out cell line and the cell line treated with U0126 and YH16899. The above results indicate that the KRS dependent dissemination of cells from the spheroid of HCT116 cultured in a three-dimensional collagen gel environment was closely related to ERK activity and paxillin expression.

### Example 9: Expression patterns of signaling activators according to the expression of KRS in the spheroidically aggregated cells cultured in a three-dimensional collagen gel environment

### <9-1> Protein levels of signaling activators according to the expression of KRS in the spheroidically aggregated cells cultured in a three-dimensional collagen gel environment

Since it was confirmed earlier that KRS played an important role in ERKs phosphorylation and paxillin expression and activation in relation to cell-ECM adhesion in HCT116 in a two-dimensional culture environment, the present inventors further investigated the ERKs activity in relation to cell-ECM adhesion in the HCT116 parental cells expressing KRS in a three-dimensional collagen gel environment by Western blotting.

Particularly, the HCT116 parental cells cultured to be spheroidically aggregated cells in a three-dimensional collagen gel environment were treated with the ERKs inhibitor U0126 at the concentration of 50 or 100 µM, followed by culture for 24 hours. Whole cell extract was collected, followed by Western blotting by the same manner as described in Example <7-2>. At this time, the primary antibodies of pY397FAK, FAK, p-ERKs, ERKs, pY118paxillin, paxillin, E-cadherin, β-catenin, KRS, α-tubulin, pAkt1/2/3, and caspase 3 were used.

As a result, as shown in Figure 7A, when ERKs were inhibited in the HCT116 parental cells cultured in a three-dimensional collagen gel environment, the phosphorylations of the cell-ECM adhesion related signaling factors FAK and paxillin were reduced at the same time, and particularly the expression of paxillin was significantly reduced. The expression of E-cadherin was also reduced (Figure 7A). The above results were consistent with the result induced by the suppression of KRS expression. That is, it was confirmed that the phosphorylation of ERKs in HCT116 parental cells was closely related to paxillin expression and phosphorylation, and also KRS played an important role in cell-ECM adhesion.

### <9-2> MRNA levels of signaling activators according to the expression of KRS in the spheroidically aggregated cells cultured in a three-dimensional collagen gel environment

Real-time PCR was performed to investigate the mRNA level of E-cadherin, one of the signaling activators, according to the expression of KRS in the spheroidically aggregated cells cultured in a three-dimensional collagen gel environment, as shown in Example <9-1>.

Particularly, the HCT116 parental cells cultured in a three-dimensional collagen gel environment were either not treated with U0126 or treated with U0126, the ERKs inhibitor, at the concentration of 50 or 100 µM, followed by culture for 24 hours. Then, real-time PCR was performed by the same manner as described in Example <2-3>. At this time, the primers used herein were hEcad-5 (SEQ. ID. NO: 5) and hEcad-3 (SEQ. ID. NO: 6), listed in Table 2.

As a result, as shown in Figure 7B, the expression level of E-cadherin mRNA was significantly reduced, compared with the control that had not been treated with the EKRs inhibitor (Figure 7B).

Therefore, it was confirmed that the KRS dependent EKRs and paxillin phosphorylations and paxillin expression were all closely related to cell-ECM adhesion and cell-cell adhesion in the HCT116 parental cells cultured in a three-dimensional environment.

### Example 10: Inhibition of cell-ECM adhesion by the expression of KRS in the spheroidically aggregated cells cultured in a three-dimensional collagen gel environment

The HCT116 parental cells expressing KRS were cultured to be spheroidically aggregated cells in a three-dimensional collagen gel environment. Then, immunofluorescence was performed to investigate whether or not the cytokines secreted from the HCT 116 parental cells and remained thereafter in the microenvironment could affect the morphology and invasion of the cells cultured above.

Particularly, collagen was added to PDMS. The HCT 116 parental cells were treated with nothing for a day or treated with the ERKs inhibitor U0126 at the concentration of 50 µM, or KRS knock out cell line was cultured. In the case of KRS knock-out cell line culture, the collagen gel containing cells was fixed in 4% paraformaldehyde at room temperature for 30 minutes, which was then treated with 30 mM glycine for 15 minutes. Then, permeabilization was induced at room temperature by using 1% triton X-100 for 30 minutes, followed by pre-treatment with 3% BSA solution for 2 hours. FITC labeled E-cadherin antibody was treated thereto at 4°C for 16 ∼ 18 hours. DAPI solution was treated thereto to stain nuclei. The stained cells were observed under confocal microscope.

As a result, as shown in Figure 8C, the expression of E-cadherin was significantly reduced in the KRS knock-out cell line, compared with the HCT116 parental cell line and the KRS over-expressing cell line, and it was even harder to observe the expression of E-cadherin in the cell-cell border. However, the adhesion between the spheroid cells was not disintegrated and still formed a solid sphere of aggregated cells in the HCT116 parental cell line and the KRS over-expressing cell line (Figure 8C). The above results indicate that KRS expression could regulate cell-cell adhesion even in a three-dimensional collagen gel environment and also affect EMT despite the effect was incomplete, and could regulate the duality of epithelial/mesenchymal cell morphology.

### Example 11: Correlation between the dissemination of KRS expressing cells and the epithelial marker cytokeratin 14 (CK14) in a three-dimensional collagen gel environment

In a three-dimensional collagen gel environment, the dissemination of cells was KRS expression-dependent, and the expressions of epithelial markers involved in cell-cell contact were reduced in the KRS knock-out cell lines. However in the KRS knock-out cell lines, the cell scattering was not observed in a two-dimensional culture environment and the dissemination was not observed in a three-dimensional collagen gel environment. So, in order to confirm whether or not the dissemination of cells from the spheroidically aggregated cells of KRS knock-out cell line in a three-dimensional collagen gel environment was attributed to the expression of epithelial marker, the cell line was cultured in a three-dimensional collagen gel environment for 24 hours. When the dissemination was observed, CK14 staining was performed and the cells were observed under confocal microscope.

Particularly, the collagen gel containing cells were distributed in PDMS, which was treated with nothing for a day (Figure 9A) or treated with U0126 (50 mM) or YH16899 (50 mM) (Figure 9B). The collagen gel containing the cultured cells was fixed in 4% paraformaldehyde for 30 minutes, which was then treated with 30 mM glycine for 30 minutes. Then, permeabilization was induced by using 0.5% triton X-100 for 30 minutes, followed by blocking with 3% BSA solution for 2 hours. The unlabeled CK14 antibody was treated thereto at 4°C for 16 ∼ 18 hours. The secondary antibody was also treated thereto at room temperature for at least 4 hours. Phalloidin staining was performed to stain F-actin, and DAPI (4',6-diamidino-2-phenylindole; blue) solution was treated thereto to stain nuclei. The stained cells were observed under confocal microscope.

As a result, in the KRS expressing cells where the dissemination was observed, CK14 expression was peculiar in those disseminated cells (Figures 9A and 9B). However, in the condition where the dissemination was inhibited such as in the condition of KRS suppression or being treated with U1026 or YH16899 despite KRS expression, CK14 expression was generally very low and was hardly observed in those cells disseminated from the spheroid (Figures 9A and 9B). Therefore, it was confirmed that the suppression of KRS expression caused down-regulation of the epithelial marker, inhibited cell scattering, and accordingly induced incomplete EMT (epithelial-mesenchymal transition). That is, the dissemination was incomplete in the KRS knock-out cell lines, suggesting that the KRS dependent dissemination was only completed in the presence of the epithelial marker.

### Example 12: The effect of KRS expression on cell morphology, migration, and invasion in the spheroidically aggregated cells cultured in a three-dimensional collagen gel environment

To investigate the effect of KRS dependent EMT related protein expression on cell morphology, migration, and invasion in the spheroidically aggregated cells cultured in a three-dimensional collagen gel environment, the cells were observed under time-lapse microscope.

Particularly, 2.5 mg/mℓ of collagen containing cells was loaded in the magnetic 4-well chamber (Live Cell Instrument, Korea) for the culture system of time-lapse microscope, and the collagen was hardened in a 37°C incubator for 30 minutes. When the collagen was fully solidified, PRMI-1640 supplemented with 10% FBS was added thereto. The magnetic chamber containing three-dimensional collagen gel and its corresponding adaptor were placed on the time-lapse microscope, followed by setting the multi-position. Then, the chamber containing three-dimensional collagen gel was observed in a 37°C 5% CO₂ incubator. Culture medium was added thereto every 6 hours in order for the collagen not to be dried. At this time, it is important to maintain the focus not to be changed.

As a result, as shown in Figure 10A, some cells were disseminated from the main cell mass over the time in the HCT116 parental cell line and the KRS expressing cell line, suggesting that there were such activities as cell migration and invasion to the surrounding area. In the meantime, the main cell mass was getting bigger but the dissemination of cells was not observed in the KRS knock-out cell line. Figure 10A presents the result of observation of the changes of cell morphology, migration, and invasion, performed every 6 hours, and at 24^{th} hour (1:00:00) and at 28^{th} hour (1:04:00) (Figure 10A).

### Example 13: Increase of TGFβ1 expression by KRS expression in the spheroidically aggregated cells cultured in a three-dimensional collagen gel environment

To investigate whether or not the result of Example 12 was attributed to the effect of autocrine of the cytokines existing in the cell microenvironment, Western blotting was performed by the same manner as described in Example <6-2> to measure the expression of TGFβ1, the most representative multifunctional and EMT inducing cytokine (Katsuno et al., 2013, Curr Opin Oncol, 25:76-84).

As a result, as shown in Figure 10B, the expression level of TGFβ1 was significantly increased over the time in the HCT116 parental cells and the KRS over-expressing cell line but was getting suppressed over the time in the KRS knock-out cell line even though the expression level of TGFβ1 was high in the early stage (Figure 10B).

Therefore, TGFβ1 was not affected in the KRS knock-out cell line, suggesting that TGFβ1 did not cause additional dissemination of cells. That is, despite it is incomplete, the EMT related factors can be regulated by the suppression of KRS expression since they have the mesenchymal cell like characteristics.

### Example 14: KRS expression dependent dissemination of cells from the spheroidically aggregated cells in a three-dimensional collagen gen environment

The effect of the microenvironment surrounding cells on the functions of the cells was confirmed in the cell line cultured in a two-dimensional culture environment and a three-dimensional culture environment (Figures 2 and 10). Therefore, it was investigated by using time-lapse microscope that if the dissemination of cells from the spheroid was dependent on KRS expression, when the spheroidically aggregated cells cultured in a three-dimensional collagen gel environment were treated with cytokines such as TNFα and TGFβ1 for 36 hours.

As a result, as shown in Figure 11A, when the spheroidically aggregated cells were treated with TNFα, the formation of invasive protrusion was increased in all of the HCT116 parental cells, the KRS over-expressing cell line, and the KRS knock-out cell line, and the dissemination of cells from the cell mass and accordingly cell migration were also observed (Figure 11A). That is, it was confirmed that the TNFα mediated invasive protrusion formation and dissemination of cells were KRS independent. However, it was presumed that such a cytokine as TGFβ1 would play an important role in the dissemination of cells (Figure 10B). So, the spheroidically aggregated cells were treated with TGFβ1. As shown in Figure 11B, the formation of invasive protrusion was not observed in the HCT116 parental cells and the KRS over-expressing cell lines when they were treated with TGFβ1, but the dissemination of cells from spheroid cells was observed. In the meantime, when the KRS knock-out cell line was treated with TGFβ1, cell migration was significantly decreased and the dissemination of cells was not observed, either (Figure 11B).

The expression levels of EMT related proteins including the epithelial marker that showed down-regulation by KRS expression were investigated. As a result, the treatment of TGFβ1 caused the increase of the epithelial marker protein in cell-cell junction, such as ZO-1 and occludin, but caused the decrease of fibronectin expression, suggesting that EMT was suppressed in the KRS knock-out cell line by TGFβ1 in the microenvironment. That is, when KRS expression was suppressed, the cells turned into incomplete mesenchymal cells (Figures 2 and 8), and thus microenvironmental factors such as the EMT causing cytokine TGFβ1 (Katsuno et al., 2013, Curr Opin Oncol, 25:76-84) could no longer affect cell dissemination, cell migration, and cell invasion.

### Example 15: Inhibition of the dissemination of cells by the suppression of ERKs in the spheroidically aggregated cells cultured in a three-dimensional collagen gel environment

As confirmed in the result of Example 14, when the HCT116 parental cells were treated with TGFβ1, the dissemination of cells from the spheroidically aggregated cells and migration thereof were observed. When the KRS knock-out cell line was treated with TGFβ1, the dissemination of cells was not observed. In the meantime, the activity of ERKs was significantly reduced in the KRS knock-out cell line, compared with the KRS expressing cell line (Figure 5). When the extracellular signal-regulated kinase (ERKs) inhibitor U0126 was treated to the HCT116 parental cells, it was presumed to be negative effect on the dissemination and migration of cells. To verify above, 50 µM of U0126, the ERKs inhibitor, was treated to the cell line, followed by observation of cell dissemination and migration by real-time live imaging of the spheroidically aggregated cells.

As a result, as shown in Figure 12, the dissemination and migration of cells were not induced when the ERKs inhibitor was treated (Figure 12). So, the dissemination of cells from the spheroid of HCT116 cells depended on the activity of EKRs.

Therefore, it was confirmed that the dissemination of cancer cells from the mass of HCT116 parental cells in a three-dimensional collagen gel environment was regulated by KRS dependent ERKs activity and paxillin expression and activity.

### Example 16: Measurement of KRS-dependent ERK activity by FRET technique

Western blotting and immunostaining are the conventional methods used to investigate the signal and activity of ERK. However, these methods have a disadvantage that only a part of cell event could be observed by snap photos. So, in this invention, the inventors tried to observe the intracellular ERK signal in live cells by FRET-basic sensor. Precisely, based on the principal that the ERK activity sensor EKAR (extracellular signal-regulated kinase activity reporter) causes conformation changes by ERK phosphorylation with increasing FRET signal, the inventors observed intracellular CFP-YFP ratio to measure ERK activity eventually (Harvey et al., 2008, Proc Natl Acad Sci USA. 2008 Dec 9;105(49):19264-9).

Particularly, HCT116 parental cells, KRS knock-out cell line, and KRS over-expressing cell line were transfected with EKAR (the extracellular signal-regulated kinase activity reporter, Harvey et al., 2008, Proc Natl Acad Sci USA. 2008 Dec 9;105(49):19264-9) for 48 hours. To observe in a two-dimensional condition, cells were distributed in a general culture dish, and 24 hours later, the cells were re-distributed (in the presence of 2% serum) in the laminin-coated dish. Two hours later, ERK activity was measured by using FRET (fluorescence resonance energy transfer) microscope. FRET images were obtained by using Nikon Ti-E inverted microscope (equipped with PFS, CoolSNAP HQ camera (Roper Scientific, Trenton, NJ), excitation and emission filter wheels) (4X4 binning mode, 200-ms exposure). All the systems were operated by using MetaMorhp software. The images obtained by intracellular FRET probe dual-emission ratio (CFP/FRET) were presented as pseudo-color images divided into 8 grades according to FRET/CFP ratio by using display (IMD) mode provided by MetMorph software.

As a result, it was confirmed that ERK phosphorylation was changed by KRS, wherein a high EKR phosphorylation signal was presented as red and a low signal was presented as blue (pseudo-color) (Figure 13A). From the statistical analysis of the results, it was confirmed that the highest EKR activity was observed in the KRS over-expressing cell line, followed by the HCT 116 parental cells and the KRS knock-out cell line in that order (Figure 13B). From the investigation of FRET signal strength, it was also confirmed that KRS expression was closely related to ERK activity.

### Example 17: The changes of paxillin expression and signaling activity in the KRS knock-out cell line transfected with ERKs

As described hereinbefore, ERKs and paxillin activation was induced in the KRS expressing parental cells and the KRS over-expressing cell line along with the dissemination of cells from spheroid cells in a three-dimensional environment. However, ERKs and paxillin activation was not induced in the KRS knock-out cell line, and neither was the dissemination of cells. So, it was further investigated whether or not the dissemination of cells from spheroid cells in a three-dimensional environment was induced by the artificially forced ERKs or paxillin over-expression in the KRS knock-out cell line.

Particularly, the KRS knock-out cell line was transfected with ERKs or paxillin gene by using lipofectamine 2000 (Life Technology, Grand Island, New York, USA) according to the manufacturer's protocol. 48 hours later, the cells were cultured in a three-dimensional collagen gel for a day, and then cell extract was obtained, followed by Western blotting.

As a result, when ERK (Ek) was expressed in the KRS knock-out cell lines shKRS2-1 and shKRS 5-4, ERK activation was induced and paxillin expression and Tyr118 phosphorylation were increased. However, FAK activity was not changed (Figure 14).

### Example 18: Formation of KRS-p67 laminin receptor conjugate and KRS-integrin α6β1 conjugate

Integrin α6β1 interacts with the extracellular ECM molecule laminin, and is also known to combine with the laminin receptor existing on the membrane (Canfield, S. M., and Khakoo, A. Y. 1999, J Immunol 163, 3430-3440). Integrin is the protein that is directly involved in intracellular adhesion signal, which is also known to regulate ERK activity (Lee, J. W., and Juliano, R, 2004, Mol Cells 17, 188-202). To investigate the interaction among KRS that induces intracellular EKR activity, integrin α6β1, and p67LR, coimmunoprecipitation was performed with the Myc labeled KRS expressing cell line. Particularly to investigate the effect of laminin, cell extract was prepared from the cells that had been replated and cultured in the laminin coated dish, followed by coimmunoprecipitation.

Precisely, the total cell extract obtained from the cells normally cultured in the medium supplemented with 10% serum (to obtain healthy cells with minimizing the effect of serum) and the other total cell extract obtained from the cells that had been replated and cultured in the laminin coated (10 µg/ml) dish supplemented with 2% serum for 2 hours were added with anti-Myc antibody respectively, followed by reaction at 4°C for at least 18 hours. The tube was picked up at 4°C, to which protein A and G (1:1 V/V) coated sepharose beads were added, followed by reaction at 4°C for 4 hours. The beads were washed with RIPA buffer, followed by boiling in 2 × SDS-PAGE sample buffer for 5 minutes. The prepared coimmunoprecipitated sample proceeded to Western blotting and the conjugation with the labeled factors was confirmed.

As a result, the interaction between myc-KRS and integrin α6, integrin β1, and p67LR was confirmed. However, when the KRS inhibitor YH16899 (Kim DG et al., Nat Chem Biol. 2014 Jan; 10(1):29-34.) was treated thereto, the interaction was suppressed. To investigate the role of laminin in the said interaction, cell extracts were re-distributed in the laminin-coated dish supplemented with 2% FBS alone, followed by coimmunoprecipitation. As a result, the interaction between myc-KRS and integrin α6, integrin β1, and p67LR was confirmed. In addition, the interaction between KRS and p67LR was confirmed even in the suspension where adhesion signal was inhibited (Figures 15A and 15B). However, the interaction between KRS and integrin α6 and integrin β1 was not observed in the suspension where adhesion signal could not be detected and only observed in the suspension where adhesion signal was detected (Figure 15A, 15B). In conclusion, the interaction between KRS and integrin α6β1 involved in cell adhesion signaling could be inhibited by the treatment of YH16899, suggesting that the interaction of KRS/p67LR/integrin α6β1 plays an important role in the activation of cell adhesion signaling activity, and at this time YH16899 can affect the interaction.

### Example 19: Paxillin expression regulated by activated ERKs, confirmed by ChIP (Chromatin Immunoprecipitation) with the cells cultured in a three-dimensional collagen gen environment

As shown in Example 17, when the KRS knock-out cell line shKRS₂₋₁ was transiently transfected with pCMV-Mock, pCMV-paxillin, and pCMV-ERK1/2 respectively and when the KRS knock-out cell line was treated with ERK1/2, paxillin expression was increased. Therefore, with the presumption that paxillin expression would be regulated by the activity of EKRs in HCT116 cell line, ChIP (Chromatin Immunoprecipitation) was performed to investigate whether or not paxillin expression was regulated by the transcription factors Elk-1 and c-Jun, known as the ERKs downstream factors.

Particularly, Western blotting was performed with the KRS knock-out cell lines cultured in a three-dimensional collagen gel environment to investigate the phosphorylation (or activation) of Elk-1 or c-Jun, the ERKs downstream transcription factor, and the phosphorylation of JNKs or p38 therein. As a result, it was confirmed that KRS mediated ERKs activation caused c-Jun activation. Chromatin fragmentation was performed with the HCT116 cells spheroidically cultured in a three-dimensional collagen gel treated with DMSO (control), the sample treated with YH16899, and the sample obtained from the KRS knock-out cell line shKRS₂₋₁ by sonication using ChIP-IT Express Enzymatic (Active motif) kit(BMS). A part of the product was left as input and the remaining chromatin was reacted with c-Jun (cell signaling Technology) or Elk-1 (Santa Cruz Biotech. Inc.) antibody in the presence of protein G magnetic beads provided from the kit, at 4°C for at least 18 hours. Upon completion of the antibody reaction, the magnetic beads were washed with ChIP buffers 1 and 2 included in the kit. After the elution of chromatin, reverse cross-linking was induced. At this time, input DNA was treated with ChIp buffer 2 and 5 M NaCl. ChIp and the input DNA sample were reacted each other at 95°C for 15 minutes. After digesting with proteinase K, the digestion was terminated by treating proteinase K stop solution. The obtained DNA proceeded to PCR using the corresponding primers listed in Table 3.

**[Table 3]**

| | |
|---|---|
| **c-Jun primer 1 F** | 5'-GAG GAC GAC CCC AGG AAA GG-3' |
| **c-Jun primer 1 R** | 5'-GTC AGC CAC TGG GTC ATC AC-3' |
| **c-Jun primer 2 F** | 5'-GCT GTA CTT GCA GAG CAG-3' |
| **c-Jun primer 2 R** | 5'-CAA CTA CCA TTT ATT GAG TGT C-3' |
| **Elk-1 primer 1 F** | 5'-GGA AGT AAT ATT AAG AGA GAT GG-3' |
| **Elk-1 primer 1 R** | 5'-CCA GGA GGA CCA TAA ATC AG-3' |
| **Elk-1 primer 2 F** | 5'-GTG ATG ACC CAG TGG CTG AC-3' |
| **Elk-1 primer 2 R** | 5'-CTC CCA GGC CTC CAG CCA C-3' |

As a result, as shown in Figure 16A, KRS expression was not involved in JNKs and p38 activation. KRS dependent ERKs activity was related to c-Jun activation and expression, among the ERKs downstream transcription factors such as Elk-1 or c-Jun. Therefore, ERKs activation in the KRS expressing cell line could cause c-Jun activation.

ChIP was also performed with the spheroidically aggregated cells cultured in a three-dimensional collagen gel environment by using c-Jun antibody. In the control, c-Jun did not combine with the nonspecific region, but combined with paxillin promoter (Figure 16B) in the specific binding sequence. However, such c-Jun binding to paxillin promoter was inhibited by the treatment of YH16899 (Figure 16C). From the above observation, it was confirmed that the phosphorylated c-Jun, the ERKs downstream factor, can affect the transcription of paxillin by combining with paxillin promoter. When treated with YH16899, c-Jun could not bind to paxillin promoter, suggesting that paxillin expression and phosphorylation was reduced by the treatment of YH16899. The inventors presumed that the transcription of paxillin would be regulated by Elk-1 known as the ERKs downstream factor, but Elk-1 antibody did not bind to paxillin promoter, confirmed by ChIP using Elk-1 antibody (Figure 16D).

As a result, it was confirmed that paxillin transcription could be regulated by the transcription factor c-Jun controlled by ERKs in the HCT116 cells cultured as spheroidically aggregated cells in a three-dimensional collagen gel environment. The decrease of paxillin expression and phosphorylation resulted from the treatment of YH16899 functioning to inhibit the conjugation between KRS and laminin receptor was attributed to the phenomenon wherein c-Jun could not bind to paxillin promoter because KRS/integrin α6β1/p67LR interaction was not complete so that ERKs activation was not induced. It was not Elk-1 but c-Jun that was involved in ERKs dependent regulation of paxillin expression in the HCT116 cells cultured as spheroidically aggregated cells in a three-dimensional collagen gel environment.

### Example 20: KRS expression dependent dissemination in other colorectal cancer cell lines and inhibition of ERKs phosphorylation and paxillin expression and phosphorylation by YH16899

To investigate whether or not the KRS expression dependent dissemination of cells from spheroid cells in the HCT116 cell line or the decrease of EKRs phosphorylation and paxillin expression and phosphorylation by the treatment of YH16899 was observed in other colorectal cancer cell lines, the following experiment was performed. Various colorectal cancer cell lines (SW620, HCT15, SNU977, KM12SM, and SNU-C5) were stably transfected with shKRS to suppress KRS expression (clones #2 and #5). Then, the dissemination was observed under time-lapse microscope (Figure 17A) or the signaling activity was investigated after the treatment of YH16899 (Figure 17B).

Particularly, the colorectal cancer cell lines SW620, HCT15, SNU977, KM12SM, and SNU-C5 (Korean Cell Line Bank, Cancer Research Institute, Seoul National University) were distributed in a three-dimensional collagen gel environment as spheroids, followed by time-lapse imaging 6 hours later or followed by normal culture in a two-dimensional environment supplemented with 10% serum for 2 days. Then, lysis buffer (50 mM Tris-HCl, pH7.4, 1% NP-40, 0.25% sodium deoxycholate, 150 mM NaCl, 1 mM EDTA) was added thereto, followed by lysis to obtain cell lysate. The protein in the obtained cell lysate was quantified, followed by Western blotting with the labeled factors.

As a result, like in the HCT116 cell line, the dissemination of cells from the three-dimensional spheroid cells was observed in the colorectal cancer cell line SW620. However, the dissemination was not observed in the KRS knock-out cell lines (#2, #5) and in the SW620 parental cells treated with U0126 or YH16899 (Figure 17A). When YH16899 was treated to various colorectal cancer cell lines, the decrease of the phosphorylations of FAK, ERKs, and paxillin, and the inhibition of the expressions of E-cadherin and paxillin were achieved without inducing apoptosis (Figure 17B). Therefore, the inhibition by YH16899 in various colorectal cancer cell lines is not just a specific phenomenon observed in some specific cell lines but is rather general phenomenon among various colorectal cancer cell lines. Accordingly, it was confirmed that YH16899 was a general inhibitor that could suppress various colorectal cancer cell lines.

### Example 21: Inhibition of ERKs phosphorylation and paxillin expression and phosphorylation by YH16899 in HCT116 cell line

The dissemination observed in the HCT116 cell line plays an important role in ERKs activation and paxillin expression and activation. Based on that, it was further investigated the expressions of focal adhesion molecule and epithelial marker according to the treatment of the EKR inhibitor U0126 (Figure 18A) and the KRS inhibitor YH16899 (Figure 18B) in a three-dimensional collagen gel.

Particularly, the HCT116 parental cells cultured in a three-dimensional collagen gel environment was treated with U0126 at the concentrations of 50 and 100 µM or YH16899 at the concentrations of 50 and 100 µM for a day. SDS, Na₃O₄V, and protease inhibitor cocktails (GenDepot) were added to lysis buffer, followed by reaction at 4°C for at least 1 hour. Centrifugation was performed at 4°C, at 13000 rpm, for 30 minutes. The supernatant was transferred into a new microcentrifuge tube. 4 x SDS-PAGE sample buffer was added thereto, followed by boiling at 100°C for 5 minutes. The extract was quantified by Western blotting using α-tubulin antibody (Sigma). Other protein expressions and phosphorylations were also measured by Western blotting.

As a result, the phosphorylation of each focal adhesion molecule such as FAK, ERKs, and paxillin was suppressed, and the expressions of the epithelial markers E-cadherin and β-catenin were also reduced (Figures 18A and 18B). Therefore, it was confirmed that the inhibition of the expressions and activations of ERKs and paxillin by the treatment of U0126 or YH16899 could cause the suppression of the dissemination of the HCT116 parental cells in a three-dimensional collagen gel.

### Example 22: Integrin α6 playing an important role in cell adhesion dependent ERKs activation in KRS expressing HCT116 parental cells

It was confirmed in the above Examples that ERKs activation in the HCT116 parental cells was closely related to the interaction of KRS, p67LR, and integrin α6β1 (Figure 15), but not related to FAK (Figure 5). So, the present inventors tried to investigate whether or not the cell adhesion dependent FAK activation (ERKs activation caused by Tyr925 phosphorylation mediated by c-Src after the phosphorylation of Tyr397: Lee JW and Juliano R. Molecules and Cells. 2004 Apr 30;17(2):188-202) could cause ERKs activation. That is, the interaction between the phosphorylation of Tyr397 and Tyr925 of FAK was investigated when ERK activation was inhibited by integrin α6 conjugation with the corresponding antibody so as to intervene the conjugation with extracellular matrix.

Particularly, HCT116 parental cells were replated in the laminin pre-coated culture dish as a suspension in the presence of 2% serum. At this time, the cells were reacted in advance with the normal IgG (Immunoglobulin, antibody control) or integrin α6 antibody for 1 hour, followed by replating. 2 ∼ 24 hours after the replating, cell extract was prepared, followed by Western blotting.

As a result, approximately 24 hours later, ERKs activation and paxillin phosphorylation were induced according to cell adhesion in the HCT116 cells cultured in a two-dimensional environment in the laminin coated dish, but were inhibited when the cells were pre-treated with integrin α6. However, FAK phosphorylation was not affected and maintained as normally as usual (Figure 19A). The above results indicate that integrin α6 plays an important role in KRS dependent ERKs activation, but does not need FAK phosphorylation as a mediator. Therefore, it was confirmed that ERKs activation, paxillin expression and activation, and further the dissemination of cells from spheroid cells in a three-dimensional collagen gel were related neither to FAK activation nor Tyr925 phosphorylation.

### Example 23: Activated FAK or over-expression of WT FAK in KRS knock-out cell line confirmed not to relate to ERKs and paxillin expressions and the dissemination in a three-dimensional collagen gel

As described in the above Examples, the dissemination observed in HCT116 cells was related to ERKs activation and ERKs activation dependent paxillin expression and activation. So, the present inventors tried to investigate whether or not the cell adhesion dependent FAK activation (ERKs activation caused by Tyr925 phosphorylation mediated by c-Src after the phosphorylation of Tyr397: Lee JW and Juliano R. Molecules and Cells. 2004 Apr 30;17(2):188-202) could cause ERKs activation and induce KRS expression dependent dissemination.

Particularly, HCT116 parental cells were infected with Ad-HA-control virus or dead form adenovirus wherein Ad-HA-R454 FAK kinase activity was eliminated, or the KRS knock-out cell line was infected with Ad-HA control virus, Ad-HA ΔN(1-100) FAK (activated FAK; Lim ST et al., Molecular Cell 2008 Jan 18;29(1):9-22), or Ad-HA-FAK WT (wild-type) virus, followed by Western blotting to measure the expressions and phosphorylations of the said molecules (Figure 19B). The spheroidically aggregated cells were obtained, which were distributed in a three-dimensional collagen gel, followed by observation under time-lapse microscope.

As a result, it was confirmed that despite the non-active (kinase activity was knocked-down) R454 FAK mutant was expressed in the KRS expressing parental cells, the dissemination of cells from spheroid cells was smoothly induced therein, like in the parental cells infected with the control virus. In the meantime, even though active FAK or wild type FAK was over-expressed in the KRS knock-out HCT116 cell line, the suppressed dissemination was not able to be recovered (Figures 19C and 19D). In the HCT116 shKRS cell line wherein the activated mutant L37A FAK (Jung et al., J Cell Sci. 2012 Dec 15; 125(Pt 24): 5960-73) was expressed, FAK activation did not cause ERKs activation (Figure 19E).

### Example 24: Incomplete improvement of ERKs and paxillin expressions and signaling activities in the KRS knock-out cell line transfected with pCMV-Mock, pCMV-paxillin, or pCMV-ERK1/2 or co-transfected with paxillin and ERK and investigation of dissemination in a three-dimensional collagen gel

As described in the above Examples, the dissemination observed in the HCT116 cell line closely relates to ERKs activation and paxillin expression and activation. So, to investigate whether or not the dissemination of cells from spheroid cells in a three-dimensional collagen gel could be induced by recovering the expression of ERKs and the expression and activity of paxillin in KRS knock-out cell line, the following experiment was performed. Stable cell lines were first prepared by treating paxillin (Pax clones) or EKRs (ERK clones) alone or paxillin and ERKs together to KRS knock-out cell line. Western blotting was performed to select those clones that demonstrated excellent ERKs and paxillin activities (Figure 20A). The selected clones were cultured to form spheroids. The spheroidically aggregated cells were distributed in a three-dimensional collagen gel, wherein the dissemination of cells was observed under time-lapse microscope for 28 hours.

Particularly, the KRS knock-out cell line was transfected with respectively pCMV-Mock, pCMV-paxillin, and pCV-ERK1/2 to establish stable cell lines. The labeled factors were confirmed by Western blotting (Figure 20A). Those cells displaying the successful expression of the transfecting molecule were selected to form spheroids. The spheroidically aggregated cells were distributed in a three-dimensional collagen gel, wherein the dissemination of cells was observed under time-lapse microscope for 28 hours (Figure 20B).

As a result, the KRS knock-out HCT116 cell line displayed either paxillin or ERK1/2 activity or both in a three-dimensional collagen gel, confirming the dissemination (Figure 20B). In particular, when ERK1/2 was treated to the KRS knock-out cell line, paxillin expression was increased (Figure 20A). Therefore, it was expected that paxillin expression could be regulated by ERKs in HCT116 cell line. The inhibition of the dissemination according to the suppression of KRS expression was confirmed to be attributed to the inhibition of ERKs and paxillin activities.

### Example 25: Correlation among KRS, paxillin, and ERKs expressions in human colon tumor tissues

Western blotting and immunohistochemical staining were performed to investigate the correlation among KRS, paxillin, and ERKs expressions in Korean colon cancer patient tissues and the corresponding normal tissues.

Particularly, Western blotting (Figure 21A) and immunohistochemical staining (Figure 21B) were performed with the tissue extract or tissues obtained from human colon tumor tissues including clinical tissues diagnosed with Grade II and III cancer to investigate paxillin, p-ERKs, and KRS. As a result, positive interaction in their invasive cancer cell margin was confirmed. Immunohistochemistry is the method for visualization of the antigen in cells or tissues. In a real cancer tissue, various cell types are observed. So, various protein expression sites and levels and interaction therein can be understood by measuring various antigen expression sites and levels in a specific cell type or tissue by immunohistochemistry.

The expressions of paxillin, p-ERKs, and KRS in human colon tumor tissues were measured by DAB staining, leading to the measurement of positive sites and positive reaction of each protein in human colon tumor tissues. It was observed that KRS expression and paxillin expression were high in the same region in grade II or III colon cancer patient tissue samples. Positive region of p-ERK and positive regions of KRS and paxillin were also consistent (Figures 21A and 21B). Therefore, as described hereinbefore, KRS, pERKs, and paxillin expression regions in the clinical colon cancer tissues were all positively related to the invasive cancer cell margin, suggesting that KRS dependent ERKs activity and paxillin expression and activity thereby play an important role in KRS mediated metastasis. It is therefore believed that cancer metastasis can be suppressed by inhibiting such factors in the early stage of cancer and the dissemination analysis system in a three-dimensional collagen gel can be very helpful for the development of a cancer metastasis inhibitor.

Those skilled in the art will appreciate that the conceptions and specific embodiments disclosed in the foregoing description may be readily utilized as a basis for modifying or designing other embodiments for carrying out the same purposes of the present invention. Those skilled in the art will also appreciate that such equivalent embodiments do not depart from the spirit and scope of the invention as set forth in the appended Claims.

## Claims

1. A method for screening a cancer metastasis inhibitor comprising the following steps:
1) culturing a cancer cell line or aggregated cancer cells wherein lysyl-tRNA synthetase (KRS) is regulated to be expressed or unexpressed in a three-dimensional environment or in the environment surrounded by extracellular matrix;
2) treating specimens to the cancer cell line or the aggregated cancer cells of step 1);
3) analyzing the activity of KRS in the cancer cell line or the aggregated cancer cells of step 2); and
4) selecting the specimens that have been confirmed to inhibit KRS activity in step 3).

2. The method for screening a cancer metastasis inhibitor according to claim 1, wherein the three-dimensional environment in step 1) is an aqueous environment or a collagen surrounding environment.

3. The method for screening a cancer metastasis inhibitor according to claim 1, wherein the cancer cell line or aggregated cancer cell of step 1) is a metastatic cancer or a metastasis inducible cancer.

4. The method for screening a cancer metastasis inhibitor according to claim 3, wherein the metastatic cancer is preferably selected from the group consisting of colorectal cancer, breast cancer, liver cancer, stomach cancer, colon cancer, bone cancer, lung cancer, pancreatic cancer, head/neck cancer, uterine cancer, ovarian cancer, rectal cancer, esophageal cancer, small bowel neoplasm, anal cancer, colon carcinoma, fallopian tube carcinoma, endometrial carcinoma, uterine cervical carcinoma, vaginal carcinoma, vulva carcinoma, Hodgkin's disease, prostatic cancer, bladder cancer, kidney cancer, ureter cancer, renal cell carcinoma, renal pelvic cancer, and central nervous system tumor

5. The method for screening a cancer metastasis inhibitor according to claim 1, wherein the specimen of step 2) is preferably selected from the group consisting of peptide, protein, non-peptide compound, antibody, antibody fragment, active compound, fermented product, cell extract, plant extract, animal tissue extract, and blood plasma.

6. The method for screening a cancer metastasis inhibitor according to claim 1, wherein the KRS activity in step 3) is the activity in cancer cells preferably selected from the group consistiing of cell-cell adhesion of cancer cells, cell-extracellular matrix (ECM) adhesion, cell scattering, wound-healing, changing the activity of cell adhesion signaling factors, c-Jun/paxillin promoter binding, and the dissemination of cells from spheroid cells.

7. The method for screening a cancer metastasis inhibitor according to claim 6, wherein the cell-cell adhesion induces the changes in expression or the location of expression of the epithelial marker selected from the group consisting of ZO-1, occuludin, CK14, β-catenin or E-cadherin, or the mesenchymal marker selected from the group consisting of fibronectin, twist, vimentin, α-SMA (smooth muscle actin), snail-1, Slug or N-cadherin, or cell scattering.

8. The method for screening a cancer metastasis inhibitor according to claim 6, wherein the cell-extracellular matrix adhesion induces the changes in the strength of cell-extracellular adhesion, cell spreading, focal adhesion and its failure, actin-reconstruction, and the expression or the location of expression of phosphorylated FAK, c-Src, EKRs, or paxillin.

9. The method for screening a cancer metastasis inhibitor according to claim 6, wherein the cell adhesion signaling activity induces the decrease or changes in the expression or phosphorylation of FAK, c-Src, ERKs, c-Jun, or paxillin, or the changes of integrin α6 conjugation.

10. The method for screening a cancer metastasis inhibitor according to claim 1, wherein the analyzing the activity of KRS in step 3) is performed by the method selected from the group consisting of Western blotting, real-time PCR, co-immunoprecipitation, ChIP (chromatin immunoprecipitation), FRET, immunofluorescence, and immunohistochemistry.

11. A method for monitoring the dissemination of cancer cells from cancer cell line or aggregated cancer cells, the epithelial-mesenchymal transition, the migration, invasion and metastasis of cancer cells, and the expression and activity of the related signaling factors, comprising the following steps:
1) culturing a cancer cell line or aggregated cancer cells wherein lysyl-tRNA synthetase (KRS) is regulated to be expressed or unexpressed in a three-dimensional environment or in the environment surrounded by extracellular matrix;
2) analyzing the activity of KRS in the cancer cell line or the aggregated cancer cells of step 1); and
3) analyzing the level of metastasis of the cancer cell line or the aggregated cancer cells based on the analyzed KRS activity of step 2).

12. The method for monitoring the dissemination of cancer cells according to claim 11, wherein the three-dimensional environment in step 1) is an aqueous environment or a collagen surrounding environment.

13. The method for monitoring the dissemination of cancer cells according to claim 11, wherein the cancer cell line or aggregated cancer cell of step 1) is a metastatic cancer or a metastasis inducible cancer.

14. The method for monitoring the dissemination of cancer cells according to claim 11, wherein the KRS activity in step 2) is the activity in cancer cells preferably selected from the group consistiing of cell-cell adhesion of cancer cells, cell-extracellular matrix (ECM) adhesion, cell scattering, wound-healing, changing the activity of cell adhesion signaling factors, c-Jun/paxillin promoter binding, and the dissemination of cells from spheroid cells.

15. The method for monitoring the dissemination of cancer cells according to claim 14, wherein the cell-cell adhesion induces the changes in expression or the location of expression of the epithelial marker selected from the group consisting of ZO-1, occuludin, CK14, β-catenin or E-cadherin, or the mesenchymal marker selected from the group consisting of fibronectin, twist, vimentin, α-SMA (smooth muscle actin), snail-1, Slug or N-cadherin, or cell scattering.

16. The method for monitoring the dissemination of cancer cells according to claim 14, wherein the cell-extracellular matrix adhesion induces the changes in the strength of cell-extracellular adhesion, cell spreading, focal adhesion and its failure, actin-reconstruction, and the expression or the location of expression of phosphorylated FAK, c-Src, EKRs, or paxillin.

17. The method for monitoring the dissemination of cancer cells according to claim 14, wherein the cell adhesion signaling activity induces the decrease or changes in the expression or phosphorylation of FAK, c-Src, ERKs, c-Jun, or paxillin, or the changes of integrin α6 conjugation.
